# EUROPEAN PATENT SPECIFICATION

(11) **EP 3 532 076 B1**
(45) Date of publication and mention of the grant of the patent: **17.03.2021**
(21) Application number: 17800414.9
(22) Date of filing: 27.10.2017
(51) Int. Cl.: A61K 35/15, A61K 39/395, A61K 45/06, C12N 5/0784, A61B 18/00, A61B 18/14, A61B 18/18, A61B 17/32, A61B 18/02, A61K 39/00, A61N 5/10, A61P 35/00, C07K 16/28

(54) **IMMUNOTHERAPEUTIC TREATMENTS FOR TUMOURS**
IMMUNTHERAPEUTISCHE BEHANDLUNGEN VON TUMOREN
TRAITEMENTS IMMUNOTHÉRAPEUTIQUES POUR DES TUMEURS

(30) Priority: 28.10.2016 GB 201618291
(43) Date of publication of application: 04.09.2019
(73) Proprietor: Vestlandets Innovasjonsselskap AS, 5006 Bergen (NO)
(72) Inventor: GJERTSEN, Bjørn Tore, 5006 Bergen (NO); KALLAND, Karl-Henning, 5006 Bergen (NO); ØYAN, Anne Margrete, 5006 Bergen (NO)
(74) Representative: Dehns
(86) International application number: PCT/EP2017/077698
(87) International publication number: WO 2018/078145

(56) References cited:
- WO-A1-2013/147642
- WO-A1-2016/146035
- WO-A2-2006/095330
- WO-A2-2016/100561
- US-A1- 2005 214 268
- M DEN BROK ET AL: "Efficient loading of dendritic cells following cryo and radiofrequency ablation in combination with immune modulation induces anti-tumour immunity", BRITISH JOURNAL OF CANCER, vol. 95, no. 7, 5 September 2006 (2006-09-05), pages 896-905, XP055350553, GB ISSN: 0007-0920, DOI: 10.1038/sj.bjc.6603341
- HURWITZ ARTHUR A ET AL: "Prostate cancer: advances in immunotherapy.", BIODRUGS : CLINICAL IMMUNOTHERAPEUTICS, BIOPHARMACEUTICALS AND GENE THERAPY 2003, vol. 17, no. 2, 2003, pages 131-138, XP9502596, ISSN: 1173-8804

## Description

The present invention provides a novel immunotherapeutic treatment for tumours. The treatment is immunotherapeutic in the sense that the treatment involves the administration of dendritic cells (DCs) to a subject following ablation of a tumour in order to induce and enhance an immune response against the cells of the tumour and the subsequent regulation of that immune response with an immune cell checkpoint inhibitor. More specifically, the treatment is a method which comprises the at least partial ablation of a tumour and the subsequent administration of immature autologous DCs and an immune cell checkpoint inhibitor to the site of the ablated tumour or part thereof. The administration of the immune cell checkpoint inhibitor may be directly to the site of the ablated tumour or part thereof and may proceed, precede or occur simultaneously with the administration of the DCs. The treatments of the invention may be associated with fewer adverse side effects than treatments with immune cell checkpoint inhibitors alone.

For more than two centuries, vaccination has been used successfully to prevent numerous infectious diseases. Vaccination consists of introducing ex vivo processed antigenic material that mimics an actual pathogen into a human subject, resulting in activation of the host's immune system against that specific pathogen.

In the case of infectious diseases, vaccines are typically employed as prophylaxis against a specific pathogen-associated disease. Upon exposure to the vaccine material, the host immune system will expand T lymphocyte populations specific to the introduced antigen/epitope(s), which in turn will revert to memory T lymphocytes that will expand upon re-exposure to the pathogen.

Cancer vaccines can be either prophylactic - for instance the recently approved vaccines against the cervical cancer-associated human papilloma virus (HPV) (Gardasil™, Merck & Cervarix™, Glaxo-Smith-Kline™) - or therapeutic. In theory, prophylactic vaccines against cancer exert their effect via the same mechanism as infectious disease vaccines, and should function optimally against cancers of viral origin. Therapeutic vaccines, on the other hand, must be capable of stimulating the host immune system to eradicate (or arrest) deposits of clinically significant cancer. Several therapeutic prostate cancer vaccines are currently being evaluated in clinical trials, including autologous dendritic cells pulsed with prostatic acid phosphatase (Sipuleucel-T™, Dendreon Corp.); whole allogeneic cell lines transfected to secrete GM-CSF (GVAX™, Cell GeneSys); and recombinant attenuated vaccinia virus engineered to express prostate-specific antigen and three immune costimulatory molecules, ICAM-1, B7.1, and lymphocyte function-associated antigen-3 (Prostvac™ Therion Biologics Corp.™). Several recent review articles discussed a variety of therapeutic vaccine approaches to metastatic prostate cancer (Anguille et al., 2014, The Lancet Oncology 15, e257-e267; Laborde et al., 2014, Frontiers in Immunology 5, 147; Marrari et al., 2007, Cancer Immunology, Immunotherapy, CII 56, 429-445; Sonpavde et al., 2007, Urologic Oncology 25, 451-459). In all cases, therapeutic vaccines are designed to stimulate an adaptive, antigen-specific immune response against cancer-associated protein (antigen) such that a cytotoxic lymphocyte-mediated response occurs against the established tumour.

The antigen comprising the immune target of the vaccine, and therefore the agent introduced into the human subject, typically takes the form of (i) "naked" proteins or peptides with or without adjuvant; (ii) protein expressed by viral vectors and introduced as viral particles; (iii) whole cancer cells, typically allogeneic, expressing a wide range of possible antigen; or (iv) recombinant or autologous proteins loaded into antigen presenting cells such as dendritic cells.

The ideal antigen for cancer vaccine therapy is a protein with specific expression only in cancer cells. However, cancer-exclusive antigens are not known to exist beyond mutations of physiological proteins such as p53 or CDK4. Indeed most tumour-associated antigens are also expressed in healthy tissues, albeit at lower levels, e.g. prostate-specific membrane antigen (PSMA) and prostate-specific antigen (PSA) which are expressed by prostate epithelial cells as well as prostatic carcinoma cells. Thus, although single antigen vaccine approaches are widely deployed against infectious disease targets, it has become increasingly clear to some investigators that multivalent (multi-antigen/ multi-epitope) approaches may be superior to monovalent ones in cancer immunotherapy, in terms of the specificity of the resultant immune response. Several recent studies detail "peptide cocktails", using a combination of known antigenic epitopes - e.g. from PSA, PSMA, PAP, PSCA (prostate stem cell antigen), in the case of prostate cancer (Anguille et al., 2014, *supra*)*.* Given the demonstrable humoral and cell-mediated immune responses directed against these HLA-restricted epitopes, it can convincingly be demonstrated that a "broader" immunologic response can be achieved with multivalent approaches than with monovalent ones. Whether the broader antigen-specific response will result in clinical responses greater than those observed in single-antigen approaches still awaits further investigation.

In addition to recombinant multi-antigen approaches, some investigators have described extraction of protein from autologous tumour as a source of multi-antigen therapeutic vaccine material - largely for introduction into dendritic cells (Chang et al., 2002, Clinical Cancer Research: an official journal of the American Association for Cancer Research, 8, 1021-1032.; Geiger et al., 2001, Cancer Research 61, 8513-8519). Although not reported in the prostate cancer literature, resected gross tumour specimen can be used as a source of multiple antigens, following acid elution, for example. Soluble autologous tumour antigen thus obtained represents a rich source of vaccine material for loading, or pulsing, into dendritic cells and other APC for vaccine strategies. However, given the nature of most primary and recurrent cancers in the prostate, where selective resection of cancer is rarely feasible given its often diffuse distribution amongst normal glandular elements, such an approach is difficult in practice where prostate cancer is concerned.

Whole cell vaccination using attenuated prostate cancer cell lines LNCaP and PC-3, transfected to secrete the cytokine GM-CSF (GVAX™, Cell GeneSys™), has been under extensive study in the U.S. (Small et al., 2007, Clinical Cancer Research: an official journal of the American Association for Cancer Research, 13, 3883-3891). Given the wide antigenic repertoire expressed by whole prostate cancer cells, one expects a potentially robust immune response against such a therapeutic vaccine. The extent of the antigenic homology between allogeneic, laboratory-maintained cell lines and clinical metastatic cancers is unknown, however, and thus the degree of the clinical response to be expected may range widely.

By whatever means the introduction of the antigen target is undertaken, the desired result of therapeutic vaccine therapy is tumour rejection, and the major effector mechanism of tumour rejection is the cell-mediated (T-cell) arm of the immune system. The recognition of the tumour antigen by the T-cell is required to stimulate an anti-tumour immune response. This T-cell recognition of antigen requires the formation of a complex comprised of: 1) the major histocompatibility complex (MHC); 2) the T-cell receptor (TCR); and 3) a short segment of intracellularly processed antigen enclosed in the MHC molecule.

The initiation of the T-cell responses requires the involvement of specialized cells known as antigen-presenting cells (APCs). Dendritic cells (DCs) are the most effective antigen-presenting cells known. DCs are bone marrow-derived cells that can phagocytose and process whole cells or proteins, migrate to the lymph nodes, and express high levels of MHC and costimulatory molecules required for T-cell activation. DCs are able to aggregate T-cells at their surface. They are the only antigen-presenting cells known to be capable of inducing primary responses in naive T-cells.

DCs deliver a signal in response to the binding of the TCR by the antigenic peptide enclosed in the MHC as well as generate stimulatory signals that are required to complete the activation sequence. These co-stimulatory signals occur mainly through the CD80/86:CD28 or CD40:CD40L pathways. Activation of T cells requires two different signals: (1) recognition of antigen/MHC by TCR and (2) co stimulatory signals. In the absence of these co-stimulatory signals, activation is terminated and the T-cell is rendered anergic. DCs are the efficient and critical link that provides both signals for an effective response between antigen and T-cell and elicitation of an immune cell activation.

Following acquisition of antigen and the development of the mature phenotype - wherein costimulatory molecules are maximally expressed - DCs develop their capacity to migrate to the lymph nodes where T-cell contact and activation generally occurs.

The ultimate goal of cancer vaccine therapy is the delivery of tumour-associated antigens into an APC population in such a way as to promote effective induction of the cytotoxic T-cell (CTL) response against tumour cells bearing the target antigen(s). As mentioned above, several attempts are being made to design vaccine therapies based on these principles.

Two clinical trials employing DC as cellular adjuvants in vaccine therapy of advanced prostate cancer (Dendreon's Sipuleucel-T and Northwest Biotherapeutics' DC-VAX-Prostate) have relied on DC loaded with specific recombinant antigen - PAP and PSMA, respectively - and induced to a mature phenotype prior to either intravenous or intradermal administration. Results from these and other DC immunotherapy trials have yielded promising data as far as specific immune responses generated, clinical responses noted, and minimal toxicity associated with therapy of metastatic prostate cancer (Higano et al., 2009, Cancer 115, 3670-3679; Quinn et al., 2014, Expert Review of Anticancer Therapy 14, 51-61; Ragde et al., 2004, The Journal of Urology 172, 2532-2538).

A controversial - yet increasingly compelling - development in the field of tumour immunology in recent years has been the acknowledgement that the adaptive immune system, while the primary mediator of tumour rejection, may also play a protective role in the growth of human cancers.

Regulatory T cells (TReg) are a subset of T cells that have the capacity to mediate active suppression of other T cells. TReg cells negatively control the activation and function of self-reactive T cells and sustain unresponsiveness or tolerance to self antigens. Abnormality in the number and function of these cells can be a chief cause of autoimmune and other inflammatory diseases. Although much of the initial attention focused on the role of TReg cells in controlling self-reactivity, there is growing evidence that TReg cells have a significant impact on the host response to cancer.

In healthy humans, most naturally occurring TReg cells are CD4+ T cells that constitutively express high level of the CD25 molecule (IL-2 receptor α-chain), representing 1-2% of circulating CD4+ T cell population in blood. In addition, most of these cells constitutively express other molecules such as FOXP3, CTLA4 and GTIR (glucocorticoid-induced tumour necrosis factor [TNF] receptor) which are involved in the TReg suppression mechanisms. Cancer patients have elevated numbers of TReg cells in peripheral blood, lymph nodes and tumour microenvironment, and high TReg frequencies are associated with reduced survival. These TReg cells can inhibit antigen-specific and allogeneic T cell activation in vitro and in vivo. It has also been demonstrated that CD4+ CD25+ TReg populations increase beyond baseline levels following initial depletion prior to cancer vaccination therapy in experimental models and in human subjects. In tumour bearing mice, CD4+ CD25+ TReg cells suppress tumour-associated antigen-specific immunity. Depletion of TReg cells in these mice alone or in combination with other immunotherapeutic approaches have resulted in improved tumour regression and survival.

In contrast to the approach taken by the Sipuleucel-T vaccine and other therapeutic DC-based anti-cancer vaccines, where DCs are exposed to one single constituent of the target cancer, are allowed to mature ex vivo, and thereafter are infused intravenously into the patient, the present invention is based in part on the principle that cancer cells, due to the cancer hallmark of genomic instability and increased mutation rate, will generate tumour neoantigens, which are believed to represent a more effective class of anti-tumour targets, and by exposing immature DCs to such antigens in situ (where such antigens will have intact tertiary structure and post-translational modifications) a more effective anti- tumour response will be generated that is tailored to the target tumour and, significantly, any metastases thereof.

This strategy has multiple advantages over previous protocols. It inherently exploits tumour neoantigens and any other tumour associated antigens on a personalised basis without having to rely on extensive next generation sequencing and peptide synthesis based upon computer based epitope predictions. It also deals inherently with the formidable challenge of cancer cell heterogeneity caused by mutations and transcriptional re-programming since the immune response is directed against whatever tumour cells are present, both in the primary tumour and any secondary growths.

Moreover, ablated tissue is largely killed by necrosis, and necrotic tissue is thought to be favourable for efficient DC maturation and (neo)antigen presentation and T cell priming compared to living tissue or apoptotic cells, in part on account of the proinflammatory cytokines which are released (e.g. (TNFα TGFβ, IL-1, IL-6, IL-10, IL-12, and IL-18)

The inventors have further recognised the role TReg cells play in dampening the development of the anti-tumour immune response mediated by immature DCs exposed to an at least partially ablated tumour and have determined that said immune response would be enhanced if an immune cell checkpoint inhibitor is administered together with the DCs to the site of the ablated tumour. Additionally, immature DCs may mature in two different directions; one direction is to a role in immune tolerance to foreign antigens, the other direction is a role in promoting an immune response against what is sensed as "foreign". The administration of an immune cell checkpoint inhibitor is believed to shift the developmental switch of DCs from tolerance and towards an anti-tumour immune response. It is however not clear from the art whether or not the use of DCs together with immune cell checkpoint inhibitors, e.g. during local co-administration to an at least ablated tumour, would have toxic effects on the DCs thus limiting the effectiveness of any anti-tumour response. The inventors have now shown that DCs do not show vulnerability to high local concentration of immune cell checkpoint inhibitors. Moreover, it was also not clear from the art whether or not tumour ablation and local administration of DCs followed by local administration of immune cell checkpoint inhibitors would cause significant surrounding tissue damage and thereby result in adverse surgical side effects like infection, fistula formation and similar local anatomical deformations. The inventors have now shown that, surprisingly, tumour ablation, e.g. by cryoablation, followed by local use of DCs together with immune cell checkpoint inhibitors is surprisingly well tolerated by patients, displaying low levels of surrounding tissue damage and thus surprisingly limited instances of infection, fistula formation and similar local anatomical deformations.

Further advantageously, by locally administering the immune cell checkpoint inhibitor to the site of the ablated tumour, lower doses may be used, thus minimising systemic side effects associated with the usually systemic administration of these agents and reducing costs. Such adverse side effects include autoimmune adverse events, e.g. autoimmune colitis, hepatitis, uveitis, hypophysitis and autoimmune skin disorders. The local administration of lower doses of immune cell checkpoint inhibitors may also help ensure the general well-being and quality of life of the subject undergoing treatment is as high as possible.

Thus the invention is as defined in the claims.

The invention provides an immune cell checkpoint inhibitor for use together with an autologous immature dendritic cell, or a precursor thereof, in a method for the treatment of a tumour in a subject, said method comprising administering
(i) the autologous immature dendritic cell, or a precursor thereof, and
(ii) the immune cell checkpoint inhibitor
to said subject subsequent to the at least partial ablation of said tumour in said subject, wherein the administration of said autologous immature dendritic cells, or a precursor thereof, and said immune cell checkpoint inhibitor is local to the site of the ablated tumour or part thereof.

Alternatively expressed the invention provides an autologous immature dendritic cell, or a precursor thereof, for use together with an immune cell checkpoint inhibitor in a method for the treatment of a tumour in a subject, said method comprising administering
(i) the autologous immature dendritic cell, or a precursor thereof, and
(ii) the immune cell checkpoint inhibitor
to said subject subsequent to the at least partial ablation of said tumour in said subject, wherein the administration of said autologous immature dendritic cells, or a precursor thereof, and said immune cell checkpoint inhibitor is local to the site of the ablated tumour or part thereof.

Alternatively expressed the invention provides an autologous immature dendritic cell, or a precursor thereof, and an immune cell checkpoint inhibitor for use together in a method for the treatment of a tumour in a subject, said method comprising administering
(i) the autologous immature dendritic cell, or a precursor thereof, and
(ii) the immune cell checkpoint inhibitor
to said subject subsequent to the at least partial ablation of said tumour in said subject, wherein the administration of said autologous immature dendritic cells, or a precursor thereof, and said immune cell checkpoint inhibitor is local to the site of the ablated tumour or part thereof.

The use of an autologous immature dendritic cell, or a precursor thereof, and/or an immune cell checkpoint inhibitor (as appropriate) in the manufacture of a medical or therapeutic product (e.g. a medicament) for the above mentioned uses is also explicitly contemplated.

In a further aspect the invention provides a product containing an autologous immature dendritic cell, or a precursor thereof, and an immune cell checkpoint inhibitor as a combined preparation for separate, simultaneous or sequential use in a method for the treatment of a tumour in a subject, said method comprising administering
(i) the autologous immature dendritic cell, or a precursor thereof, and
(ii) the immune cell checkpoint inhibitor
to said subject subsequent to the at least partial ablation of said tumour in said subject, wherein the administration of said autologous immature dendritic cells, or a precursor thereof, and said immune cell checkpoint inhibitor is local to the site of the ablated tumour or part thereof.

An "immune cell checkpoint", or in more general terms, an "immune checkpoint", is a cell signalling pathway within the cells of the immune system which negatively regulates the immune system in some way. Typically, such signalling involves a receptor-ligand interaction at the immune cell surface, usually an interaction between a cell surface localised receptor on one cell and a cell surface localised ligand on another cell. Signalling through these pathways may result a general suppression of the immune system or may suppress a specific immune response. Consequently, it will immediately be seen that immune cell checkpoints are crucial for maintaining self-tolerance and for modulating the duration and amplitude of immune responses to foreign antigens in order to minimise collateral tissue damage.

Immune cells are considered to be leukocytes, e.g. the phagocytes (e.g. monocytes, macrophages, neutrophils, DCs, mast cells), the lymphocytes (e.g. natural killer (NK) cells, T cells and B cells), eosinophils and basophils. B cells may for instance be plasma B cells or memory B cells. T cells may for instance be regulatory T cells (TReg), effector T cells (e.g. helper T (T_{H}) cells, cytotoxic T cells (T_{C}, cytotoxic T lymphocytes, CD8+ T cells)), memory T cells, natural killer T cells, mucosal associated invariant T cells and gamma delta T cells.

In certain embodiments the immune cell checkpoint which is inhibited by the inhibitor of use in the invention comprises a signal transduction pathway of a T cell, preferably a T_{C} cell or a TReg cell. In such embodiments the immune cell checkpoint inhibitor may be described as an inhibitor of a T cell immune checkpoint or an inhibitor of immunoinhibitory T cell signalling (signal transduction). Preferably such inhibitory T cell signal transduction pathways are those which are activated upon binding of receptor localised at the surface of the T cell and a ligand localised at the surface of an antigen presenting cell, e.g. a DC.

Known immune cell checkpoint receptor and ligand pairs include, but are not limited to, cytotoxic T-lymphocyte-associated antigen 4 (CTLA4 (CD152)) and CD80 (B7.1) or CD86 (B7.2); programmed cell death protein 1 (PD1 (CD279)) and PD1 ligand 1 or 2 (PDL1 (B7-H1; CD274) or PDL2 (B7-DC; CD273)); B and T lymphocyte attenuator (BTLA (CD272)) and herpesvirus entry mediator (HVEM (TNFRSF14)); killer cell immunoglobulin-like receptor (KIR) and MHC class I or II; lymphocyte activation gene 3 (LAG3 (CD223)) and major histocompatibility complex (MHC) class I or II; T cell membrane protein 3 (TIM3 (HAVcr2)) and galectin 9 (GAL9); and adenosine A2a receptor (A2aR) and adenosine. Further known immune cell checkpoint ligands include, but are not limited to, B7-H3 (CD276) and B7-H4 (B7-S1, B7x and VCTN1). The identities of the receptors for these ligands are not clear. Further known immune cell checkpoint receptors include, but are not limited to, 2B4 (CD244). The identity of the ligand(s) for this receptor is not clear.

An immune cell checkpoint inhibitor is an agent which inhibits (combats, reduces, abrogates, prevents or eliminates) signal transduction through one or more of the above pathways and hence combats (e.g. inhibits, reduces, abrogates, prevents, reverses or eliminates) the suppressive effects of one or more of said immune checkpoints on the immune system. In particular embodiments the immune cell checkpoint inhibitor is an agent which combats the activities of TReg cells or augments (enhances, stimulates, increases) the activities of T_{C} cells on the immune response induced by the autologous immature dendritic cells which are administered together with the checkpoint inhibitor in accordance with the invention.

TReg and T_{C} cell activity may be measured by any convenient means, e.g. those described in detail in Kolstad A. et al, 2015, Blood, 125(1) 82-89 and Olson BM and McNeel DG, 2013, Frontiers in Oncology, Vol 3, Article 109, the contents of which are referred to herein. In certain embodiments activity may be determined by reference to the number of and proliferation rates of said cells, e.g. in response to antigenic stimulation. The effects of an immune cells checkpoint inhibitor may therefore, in certain embodiments, be assessed by determining TReg and T_{C} cell activity (e.g. numbers thereof or proliferation rate thereof) following antigenic stimulation) in the presence and absence of the putative inhibitor.

The mechanism by which the inhibitor exerts such effects is not limited and thus inhibition may take place at any point on said pathways, e.g. at the cell surface at the receptor/ligand level, at the level of resulting gene transcription and translation or at the level of the intervening signalling cascade.

In certain embodiments the inhibitor interferes with the interaction between at least one immune checkpoint ligand and its receptor, e.g. those described above. This interference may occur through any means, e.g. through the blocking of the interaction, through the disruption of the interaction or through the depletion of the pool of ligand and/or receptor at the cell surface. These effects may be achieved, for example, with small molecule antagonists which block the relevant binding sites, competitively or non-competitively, or larger interactive macromolecules which bind to the ligand and/or the receptor and hinder their interaction, e.g. by occupying or masking the relevant binding sites. Further mechanisms include those which reduce the amount of receptor or ligand at the cell surface, e.g. gene knockout or knock down or silencing techniques and RNA interference techniques (antisense oligonucleotides, miRNA, siRNA).

In preferred embodiments the inhibitor is an affinity, e.g. immunoaffinity, macromolecule reagent which binds to, preferably specifically and with high affinity, at least one immune checkpoint ligand and/or its receptor and the binding of which to its target binding site interferes with (inhibits, reduces, abrogates, prevents, reverses or eliminates) the interaction between the receptor and its ligand.

The affinity (or immunoaffinity) macromolecule reagent may be a protein. In certain embodiments said affinity protein may comprise a portion of the amino acid sequence of a checkpoint ligand or receptor, e.g. a portion comprising the amino acid regions involved in the binding interaction between said ligand or receptor and the opposite member of the corresponding binding pair. In these embodiments the portion may be described as an isolated portion because the affinity protein does not comprise the full length amino acid sequence, or essentially all, substantially all, or the majority, of the full length amino acid sequence of the ligand or receptor from which said portion is derived. In certain embodiments this affinity (interactive) protein may be a fusion protein comprising said portion of amino acid sequence and one or more amino acid sequences which are heterologous to the ligand or receptor from which said portion is derived. Said heterologous amino acid sequences may be immunoglobulin (antibody) amino acid sequences, e.g. amino acid sequence from those immunoglobulins recited below. Said heterologous amino acid sequences will preferably be non-immunogenic or minimally immunogenic.

In other embodiments said immunoaffinity protein may be an antibody (e.g. IgA, IgD, IgE, IgG, IgM, IgY, IgW) or antigen binding fragment thereof (e.g. CDRs, Fv, Fab or Fc regions), a phage display antibody or an antibody mimetic (e.g. an affibody, an affilin, an affimer, an affitin, an alphabody, an anticalin, an avimer, a DARPin, a fynomer, a Kunitz domain peptide, a domain antibody, a nanobody, a unibody, a duocalins, a versabody or a monobody).

The term "antibody" as used herein refers to a peptide or polypeptide derived from, modelled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites" (e.g. fragments, subsequences, complementarity determining regions (CDRs)) that retain capacity to bind antigen, including (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody". Antibodies of the invention include, but are not limited to polyclonal, monoclonal, bispecific, humanized or chimeric antibodies, single chain antibodies, Fab fragments and F(ab')₂ fragments, fragments produced by a Fab expression library, anti-idiotypic (anti-Id) antibodies, and epitope-binding fragments of any of the above. The immunoglobulin molecules of the invention can be of any class (e.g. IgG, IgE, IgM, IgD, IgA, IgY and IgW) or subclass of immunoglobulin molecule.

The terms "specifically binds" or "binds specifically" (or "immunospecifically binds") is not intended to indicate that an antibody (or other affinity macromolecule reagent) binds exclusively to its intended target, although this is not excluded and would be preffered. Rather, an affinity macromolecule reagent "specifically binds" if its affinity for its intended target is typically about 5-fold greater when compared to its affinity for a non-target molecule. Suitably there is no significant cross-reaction or cross-binding with undesired substances, especially naturally occurring proteins of a healthy human or animal which are not expressed by the tumour. Preferably the affinity of the affinity macromolecule reagent will be at least about 5-fold, preferably 10-fold, more preferably 25-fold, even more preferably 50-fold, and most preferably 100-fold or more, greater for a target molecule than its affinity for a non-target molecule. In some embodiments, binding between an affinity macromolecule reagent and its target (e.g. an antibody and its epitope on an antigen) may have a "high" binding affinity of at least 10⁶ M⁻¹, e.g. at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off}/kₒₙ (k_{off} is the dissociation rate constant, kₒₙ is the association rate constant and K_{d} is the equilibrium constant. Affinity can be determined at equilibrium by measuring the fraction bound (r) of labelled ligand at various concentrations (c). The data are graphed using the Scatchard equation: r/c = K(n-r):
where
r = moles of bound ligand/mole of receptor at equilibrium;
c = free ligand concentration at equilibrium;
K = equilibrium association constant; and
n = number of ligand binding sites per receptor molecule

By graphical analysis, r/c is plotted on the Y-axis versus r on the X-axis thus producing a Scatchard plot. The affinity is the negative slope of the line. k_{off} can be determined by competing bound labelled ligand with unlabelled excess ligand (see, e.g. U.S. Pat No. 6,316,409). Antibody affinity measurement by Scatchard analysis is well known in the art, see, e.g. van Erp et al., J. Immunoassay 12: 425-43, 1991; Nelson and Griswold, Comput. Methods Programs Biomed. 27: 65-8, 1988.

In certain embodiments the antibodies of use in the invention are monoclonal antibodies, e.g. IgG, which bind to, preferably specifically and with high affinity, at least one immune checkpoint ligand and/or its receptor and the binding of which to its target binding site (epitope) interferes with the interaction between the receptor and its ligand.

In preferred embodiments the at least one immune checkpoint ligand and/or its receptor to which the affinity macromolecule reagents (e.g. antibodies) of use in the invention are targeted (i.e. bind) are selected from CTLA4, CD80, CD86, PD1, PDL1, PDL2, LAG3, B7-H3, B7-H4 , or TIM3, preferably CTLA4, PD1, PDL1, LAG3 or B7-H3, more preferably CTLA4 or PD1.

In certain embodiments the immune checkpoint inhibitor may be selected from ipilimumab (anti-CTLA4), tremelimumab (anti-CTLA4), MDX-1106 (also known as BMS-936558 and nivolumab; anti-PD1 antibody), MK3475 (also known as pembrolizumab; anti-PD1 antibody), CT-011 (anti-PD1 antibody), AMP-224 (anti-PD1 fusion protein - PDL2-Ig fusion protein), MDX-1105 (anti-PDL1 antibody), RG7446 (anti-PDL1 antibody), BMS-936559 (anti-PDL1 antibody), MGA271 (anti-B7-H3 antibody), atezolizumab (also known as MPDL3280A; anti-PDL1 antibody), avelumab (also known as MSB0010718C; anti-PDL1 antibody) and durvalumab (anti-PDL1 antibody).

In other embodiments it may be advantageous to administer more than one checkpoint inhibitor, and in particular a plurality of checkpoint inhibitors which together inhibit at least two checkpoints, e.g. two or more of the immune cell checkpoints recited above, e.g. two or more of CTLA4, CD80, CD86, PD1, PDL1, PDL2, LAG3, B7-H3, B7-H4, or TIM3, preferably two or more of CTLA4, PD1, PDL1, LAG3 or B7-H3, more preferably at least CTLA4 and PD1.

Suitable doses of the immune cell checkpoint inhibitor may vary between subjects and may be determined by the skilled person without undue burden based on the subject's physical characteristics, the characteristics of the tumour, the pharmacokinetic characteristics of the inhibitor and/or by monitoring TReg and/or T_{C} cell activity of the subject, e.g. as discussed above, or overall clinical indicators as discussed below.

In the particular case of antibody based immune cell checkpoint inhibitors, e.g. an anti-CTLA antibody or an anti-PD1 antibody, e.g. ipilimumab and tremelimumab, suitable doses for localised administration to the site if the ablated tumour or part thereof may be 0.01 to 3 mg/kg, e.g. 0.01 to 2 mg/kg, 0.01 to 1.5 mg/kg, 0.01 to 1.2, mg/kg, 0.01 to 1 mg/kg, 0.01 to 0.9 mg/kg, 0.01 to 0.8 mg/kg, 0.01 to 0.7 mg/kg, 0.01 to 0.6 mg/kg, 0.01 to 0.5 mg/kg, 0.01 to 0.4 mg/kg, 0.01 to 0.3 mg/kg, 0.01 to 0.2 mg/kg, 0.01 to 0.1 mg/kg, 0.01 to 0.05 mg/kg, 0.01 to 0.02 mg/kg, 0.02 to 3 mg/kg, 0.05 to 3 mg/kg, 0.1 to 3 mg/kg, 0.2 to 3 mg/kg, 0.3 to 3 mg/kg, 0.4 to 3 mg/kg, 0.5 to 3 mg/kg, 0.6 to 3 mg/kg, 0.7 to 3 mg/kg, 0.8 to 3 mg/kg, 0.9 to 3 mg/kg, 1 to 3 mg/kg, 1.2 to 3 mg/kg ,1.5 to 3 mg/kg or 2 to 3 mg/kg, 0.1 to 1 mg/kg, 0.2 to 0.9 mg/kg, 0.3 to 0.8 mg/kg, 0.4 to 0.7 mg/kg, 0.5 to 0.6 mg/kg, 0.2 to 0.7 mg/kg, or 0.3 to 0.6 mg/kg,. Doses of about 0.3 mg/kg, e.g. 0.1 to 0.5 mg/kg, 0.2 to 0.4 mg/kg or 0.25 to 0.35 mg/kg, or about 0.6 mg/kg, e.g. 0.4 to 0.8 mg/kg, 0.5 to 0.7 mg/kg or 0.55 to 0.65 mg/kg, may be advantageous. Any ranges which may be formed from the range endpoints recited above are explicitly contemplated. These doses may be administered as a one-time dose, e.g. at the same or substantially the same time as the DCs are administered, or may be repeated over time, in which case the above doses may be considered to be total daily doses, total weekly doses, total two week doses or total three week doses. If repeatedly administered the above may be administered as a single dose once every one, two or three weeks.

The immune cell checkpoint inhibitor is administered locally to the site of the ablated tumour or portion thereof by any convenient and routine means available in the art and typically in the form of a composition comprising said inhibitor, e.g. those described in detail below. Typically this will involve local administration (or "localised delivery", which terms are used interchangeably) of the immune cell checkpoint inhibitor to said site or its immediate vicinity, namely without administration or drug diffusion beyond that vicinity, e.g. as would be seen following systemic administration. Expressed alternatively, local administration means that effective amounts of the immune cell checkpoint inhibitor are restricted to the site of the ablated tumour or portion thereof or the immediate vicinity. The "immediate vicinity" is that volume of space from which the immune cell checkpoint inhibitor is capable of exerting an immunotherapeutic effect in accordance with the invention following local delivery. This may be further expressed as the volume of space immediately surrounding the site of the ablated tumour or portion thereof. Preferably, the immune cell checkpoint inhibitor is administered to (or its effects restricted to) the site of the ablated tumour or portion thereof. One specific embodiment of such administration may be described as intratumoural administration. In these embodiments delivery is preferably directly to the site of the ablated tumour or portion thereof or its immediate vicinity, i.e. not via a blood vessel which is connected to or which leads to the site of the ablated tumour or portion thereof or its immediate vicinity. In certain embodiments the subject will not receive an immune cell check point inhibitor by systemic administration or will not experience systemic, or at least distributed, exposure to an immune cell check point inhibitor together with the DCs during their administration in accordance with the invention, or during the course of its treatment in accordance with the invention. In other embodiments the local administration of immune cell checkpoint inhibitors together with DCs, as described above, is followed by one or more systemic administrations of immune cell checkpoint inhibitors, e.g. at least one month after the final administration of DCs.

A "dendritic cell" (DC) is an antigen presenting cell (APC) with a characteristic morphology including lamellipodia extending from the dendritic cell body in several directions. Dendritic cells are able to initiate primary, antigen-specific T cell responses both *in vitro* and *in vivo,* and direct a strong mixed leukocyte reaction (MLR) compared to peripheral blood leukocytes, splenocytes, B cells and monocytes. DCs can be derived from a number of different hematopoietic precursor cells. For a general description of dendritic cells, including their differentiation and maturation, see, e.g. Steinman, Annu Rev Immunol. 9:271-96 (1991), and Lotze and Thomson, Dendritic Cells, 2nd Edition, Academic Press, 2001.

An "immature" DC of use in accordance with the invention is a DC which is not presenting antigens, in particular a DC which has not been exposed to material which may be presented by the DC. Thus, in certain embodiments the immature DC may be in the process of maturing but has not begun presenting the antigens which it has encountered. In each context the DC may or may not have been exposed to DC maturation factors, e.g. monocyte-conditioned media (MCM), TNF-α LPS, IL1-β, and bacillus Calmette Guerin (BCG), optionally in combination with other factors like prostaglandin-E2 (PGE2), vasoactive intestinal peptide, poly-dldC, as well as mycobacterial cell wall components.

More specifically, an immature DC is a DC in which there us essentially or substantially no expression of (i.e. low levels of expression of) each of the following: the mature DC marker CD83, the costimulatory molecules CD40, CD80, and CD86, and/or the class II MHC Ag-presenting molecule HLA-DR (Vieira et. al., J. Immunol. 184:4507-4512 (2000)). In other embodiments an immature DC is a DC which is not expressing, or expressing essentially or substantially none of (i.e. low levels of expression of), one or more of the foregoing, especially CD83. In further embodiments an immature DC is a DC which is also not expressing all of TNFα, TGFβ, IL-1, IL-6, IL-10 and IL-18m. In still further embodiments an immature DC is a DC which is also not expressing any of TNFα, TGFβ, IL-1, IL-6, IL-10 and IL-18m. In more detailed embodiments an immature DC will also not express CD14, CD3, CD19, CD16+CD56 and CD66b.

The autologous immature DCs for use in accordance with the present invention may be prepared by any convenient and routine means available in the art.

DCs may, for example, be purified from whole blood, plasma or leukocyte (e.g. mononuclear cells) containing samples by removal of other defined cell populations, e.g. erythrocytes, T and B lymphocytes, natural killer cells and monocytes, by using antibodies and magnetic beads, panning or a cell sorter (Banchereau and Steinman, Nature 392:245 (1998); Freundenthal and Steinman, Proc. Natl. Acad. Sci. USA 87:7698 (1990); Steinman, Annu. Rev. Immunol. 9:271 (1991)).

It may however be more convenient and provide greater certainty of obtaining immature DCs to prepare the DCs of use in the invention from their precursor cells (or progenitor cells, which terms are used interchangeably). For instance, monocytes are among the most abundant DC precursors in blood and may be differentiated into DCs in vitro in the presence of granulocyte macrophage-colony stimulating factor (GM-CSF) and one or more additional cytokines selected from IL-4, IL-7, IL-13 and IFN-α. Methods for in vitro differentiation of monocytes into DCs in a medium including GM-CSF, IL-4 and TNF-α are described in US 5849589. The use of IL-7 to induce monocyte differentiation and DC maturation has been described, for example, by Fry and Mackall, Blood 99:3892-3904 (2002); Li, et al., Scand. J. Immunol. 51:361-371 (2000), and Takahashi, et al., Human Immunol. 55:103-116 (1997). Combinations of GM-CSF with at least IL-4 or IFN-α may be advantageous.

Other DC precursor cells include, but are not limited to, macrophage and DC progenitor (MDP), common DC progenitor (CDP), common myeloid progenitor (CMP); granulocyte and macrophage progenitor (GMP); and haematopoietic stem cells (HSCs), in particular CD34+ HSCs. However, in certain embodiments the precursor is not an embryonic stem cell (human or non-human) or derived from an embryo (human or non-human). In other embodiments the precursor is not a stem cell (human or non-human).

DC precursors, including those mentioned above, may be isolated by a variety of methods known in the art, including plating, separation on magnetic beads, tangential gel filtration, or using the Elutra™ Cell Separation System (Gambro BCT, Lakewood, Colo., USA) and identified through the analysis of well-established and well-characterised expression patterns of cell surface markers. The in vitro differentiation of such cells into immature DCs is well established and described in detail in the art and as such entirely routine.

In certain embodiments the technique used to induce DC differentiation does not include a separate maturation step in which DCs or their precursors, e.g. monocytes, are incubated in the presence of a maturation factor (e.g. monocyte conditioned media, LPS, TNF-α, IL1-β and bacillus calmette guerrin (BCG)).

Once a population of DCs has been prepared, preferably a population of sufficient number to permit administration to the subject in accordance with the invention, the population may be cryopreserved and stored ready for administration.

In certain embodiments it may be advantageous to contact the immature DCs with IFN-γ prior to administration. It has been shown that IFN-γ treated immature DC are more effective at secreting IL12 and other proinflammatory cytokines, during later T cell priming interactions which in turn leads to a more robust immune response to the antigen presented by the DC. Contact between the immature DC and the IFN-γ may occur at any time prior to maturation and antigen presentation, e.g. during or immediately after the immature DC is administered to the subject, immediately before administration, prior to cryopreservation or during or after thawing or following maturation from a precursor.

In one particular embodiment the preparation of the autologous immature DCs or monocytes of use in accordance with the invention may involve (i) leukapheresis of the subject to isolate leukocytes or mononuclear cells from the blood of the subject and/or to isolate monocytes from the blood of the subject, (ii) optionally, isolation of monocytes from the isolated leukocytes or mononuclear cells of (i), and (iii) optionally culture of said monocytes under conditions which permit differentiation of DCs therefrom (without a separate maturation step). In other embodiments the method further involves (iv) isolation of the immature DCs from the culture and, optionally, (v) cryopreservation of said autologous immature DCs.

In embodiments in which leukapheresis is used to isolate monocytes from a subject and said monocytes are differentiated into immature DCs, it may be advantageous to perform the leukapheresis about 7-28 days, e.g. about 7-25 days, 7-21 days, 7-18 days, 7-14 days, 7-10 days, 10-28 days, 10-25 days, 10-21 days, 10-18 days, 10-14 days, 14-28 days, 14-25 days, 14-21 days or 14-18 days, preferably about 14-21 days before the DCs are to be administered to the subject.

In more specific embodiments the immature DCs are contact with IFN-γ in a sufficient amount and for sufficient time to enhance secretion of proinflammatory cytokines during T cell priming treatment. The method of the invention disclosed herein may include some or all of these steps. Particular protocols for performing these steps are provided in the Examples below, however, other protocols are known in the art and may be used. In these embodiments "isolated" refers to the act of separating the target cell from the essentially all of the other cellular and preferably cell-derived matter, constituents of the sample from which isolation occurs.

The autologous immature DCs or precursors thereof are administered locally to the site of the ablated tumour or portion thereof by any convenient and/or routine means available in the art and typically in the form of a composition comprising said cells, e.g. those described in detail below. Typically this will involve local administration (or "localised delivery", which terms are used interchangeably) of the DCs or precursors thereof to said site or its immediate vicinity, namely without administration beyond that vicinity, e.g. systemic administration. Expressed alternatively, local administration means that effective amounts of the DCs or precursors thereof are, at least initially, restricted to the site of the ablated tumour or portion thereof or its immediate vicinity. The "immediate vicinity" is that volume of space from which the DCs or precursors thereof are capable of exerting an immunotherapeutic effect in accordance with the invention following local delivery. This may be further expressed as the volume of space immediately surrounding the site of the ablated tumour or portion thereof. Preferably, the DCs or precursors thereof are administered to (or their effects initially restricted to) the site of the ablated tumour or portion thereof. One specific embodiment of such administration may be described as intratumoural administration. In these embodiments delivery is preferably directly to the site of the ablated tumour or portion thereof or its immediate vicinity, i.e. not via a blood vessel which is connected to or which leads to the site of the ablated tumour or portion thereof or its immediate vicinity.

The amount of cells administered may vary between subjects and may be determined by the skilled person without undue burden based on the subject's physical characteristics, the characteristics of the tumour, the characteristics of the cells to be administered and/or by monitoring the immune response of the subject against the tumour, e.g. as discussed herein, or overall clinical indicators as discussed below.

In certain embodiments it may be found that administering a total of 10⁶ to 10⁹ cells, e.g. 5x10⁶ to 10⁹ cells, 10⁷ to 10⁹ cells, 5x10⁷ to 10⁹ cells, 10⁸ to 10⁹ cells, 5x10⁸ to 10⁹ cells, 10⁶ to 5x10⁸ cells, 10⁶ to 10⁸ cells, 10⁶ to 5x10⁷ cells, 10⁶ to 10⁷ cells or 10⁶ to 5x10⁶ cells, locally to the site of the ablated tumour or portion thereof is effective. In other embodiments it may be found that administering a total of about 7.5x10⁷ cells, e.g. 10⁷ to 5x10⁸ cells, 2.5x10⁷ cells to 2.5x10⁸ cells, 5x10⁷ to 10⁸ cells or 7x10⁷ to 8x10⁷ cells, locally to the site of the ablated tumour or portion thereof is effective. Any ranges which may be formed from the range endpoints recited above are explicitly contemplated. These doses may be administered as a one-time dose, e.g. at the same or substantially the same time as the immune cell checkpoint inhibitor is administered, or may be repeated over time, in which case the above doses may be considered to be total daily doses, total weekly doses, total two week doses or total three week doses. If repeatedly administered the above may be administered as a single dose once every one, two or three weeks.

It may be advantageous to administer the autologous immature DCs or precursors thereof immediately after tumour ablation takes place, e.g. within 5mins of ablation, or within 10mins, 15mins, 20mins, 30mins, 60mins, 90mins, 120mins of ablation, for instance because of a need to minimise operating time, to minimise adverse outcomes from the procedure and for reasons of increased efficacy. Typically, in the context of ablation procedures which induce significant temperature changes in the tumour and surrounding tissue (e.g. cryoablation) the treated area will be allowed to return to normal body temperature before DCs are administered. In other embodiments there may be a greater time between ablation and the administration of the autologous immature DCs or precursors thereof.

In these embodiments it may be possible to time the administration of the autologous immature DCs or precursors thereof to coincide with the greatest bioavailability of the cancer-specific antigens to ensure the greatest uptake and presentation of cancer-specific antigens by the autologous immature DCs or precursors thereof.

In accordance with the invention the bioavailability of antigens can be monitored using any assay format suitable for detecting a particular tumour-associated antigen or a group of antigens and the levels thereof (e.g. at the site of the ablated tumour or part thereof, or in the vicinity of said site, or in surrounding tissue or in body fluid such as blood and lymph). Suitable methods of antigen detection include, without limitation, immunoassays, which may be in ELISA format, antibody-based chemoluminescence assays, and assays measuring a bioactivity of a target tumour antigen. Suitable biomarkers for a target cancer are known or may be determined for each subject. It should not be necessary in all cases to measure the precise antigens deemed to be of most relevance to a particular treatment since release of all antigens following ablation should be proportional and the skilled person need only monitor the rise in antigen levels overall in order to determine when to administer the autologous immature DCs or precursors thereof.

The timecourse and pattern of administration of the immune cell check point inhibitor and the autologous immature DCs or precursors thereof relative to one another, i.e. their "use together" or "together with" one another is not restricted. Thus, the immune cell check point inhibitor of use in the invention may conveniently be administered before, simultaneously with or following the DCs (which term, for the sake of brevity, includes reference to the autologous immature DCs or precursors thereof of use in the invention). Conveniently the inhibitor is applied at substantially the same time as the DCs or afterwards. In other embodiments the inhibitor may conveniently be applied or administered before the DCs.

By "use together" or "together with" it is particularly meant that an effective amount of the immune cell check point inhibitor and an effective amount of the DCs are administered/applied in a manner that results in the inhibitor and the DCs contacting the site of the ablated tumour or part thereof at the same, or substantially the same, time or the DCs contacting the site of the ablated tumour or part thereof prior to the inhibitor.

In certain embodiments the inhibitor can be given (e.g. administered or delivered) repeatedly at time points appropriate for the agent(s) used or the DCs can be given (e.g. administered or delivered) repeatedly at appropriate time points. In long term treatments both the inhibitor and the DCs can be used repeatedly. The inhibitor can be applied as frequently as the DCs, or more or less frequently. The skilled person is able to devise a suitable dosage regimen. It may be advantageous in these embodiments that the first, or only, administration of DCs is prior to the first administration of the immune cell checkpoint inhibitor.

It will be seen therefore that the immune cell check point inhibitor may be applied or administered simultaneously with the DCs or sequentially. As noted above, in one embodiment the inhibitor is administered at the same or substantially the same time as the DCs, and in another embodiment it is administered after the DCs. In other embodiments however the DCs are administered separately to and after the inhibitor. Included within the scope of "substantially the same time" is application or administration of the DCs immediately or almost immediately before or after the inhibitor. The term "almost immediately" may be read as including application or administration within one hour of the previous application or administration, preferably within 30 minutes. However, the inhibitor may be applied or administered at least 1 hour, at least 3 hours, or at least 6 hours or more after the DCs. In these embodiments the inhibitor can be applied or administered with or without a further application of DCs. The DCs can also be applied or administered in a plurality of applications prior to or with the inhibitor, including as noted above, an application or administration immediately with or almost immediately after the inhibitor. In other embodiments the inhibitor may conveniently be applied or administered before the DCs, e.g. at least 1 hour, at least 3 hours, at least 6 hours before the DCs. In these embodiments the DCs can be applied or administered with or without a further application of the inhibitor. The inhibitor can also be applied or administered in a plurality of applications prior to or with the DCs, including as noted above, an application or administration immediately with or almost immediately after the DCs.

In certain embodiments the immune cell check point inhibitor is administered immediately after the DCs. In still further embodiments the immune cell check point inhibitor is administered immediately after the DCs, which in turn are administered immediately following at least partial ablation of the tumour, in particular once any temperature deviation caused by the ablation technique has normalised.

It will further be seen that "use together/together with" does not imply that the respective agents are necessarily present in the same formulation or composition, and accordingly even if used, or administered, at the same or substantially the same time, the DCs and immune cell check point inhibitor need not, indeed most likely will not, be present in the same composition or formulation, but may be administered separately. Thus "separate" use/administration includes use/administration at the same or substantially the same time, or at different times, e.g. sequentially, or at different time intervals according to the desired dosage or usage regime.

Nevertheless, embodiments in which both of the respective agents are present in the same formulation or composition, at least at the point of administration, are envisaged and may be advantageous in certain contexts.

The arrangement of the combination product described above should be construed in accordance with the entirety of the foregoing.

In these embodiments the method of the invention may be considered a method for improving or enhancing the effectiveness (or efficacy) of a DC cancer immunotherapy, said method comprising using one or more of the immune cell check point inhibitors described herein together with the autologous immature DCs or precursors thereof described herein, in particular following at least partial ablation of a tumour. Said improvement or enhancement is measured relative to effectiveness/efficacy in the absence of said inhibitor.

In these embodiments the method of the invention may be considered a method for improving or enhancing the effectiveness (or efficacy) of immune cell checkpoint inhibitor immunotherapy, said method comprising using one or more of the immune cell check point inhibitors described herein together with the autologous immature DCs or precursors thereof described herein, in particular following at least partial ablation of a tumour. Said improvement or enhancement is measured relative to effectiveness/efficacy in the absence of said autologous immature DCs or precursors thereof.

As explained above, it was not clear from the art whether or not the use of DCs together with immune cell checkpoint inhibitors, e.g. during local co-administration to an at least ablated tumour, would have toxic effects on the DCs thus limiting the effectiveness of any anti-tumour response involving the two agents. The inventors have now shown that DCs do not show toxic side effects due to immune cell checkpoint inhibitors at high local concentrations.

Tumour ablation is a process by which a tumour, or a part or portion thereof, is destroyed (sometime referred to as "lysed") by physical means and, typically, the remnants of the tumour resulting from the ablation step are left in situ. Ablation may therefore be contrasted to simple surgical excision where tumour tissue is removed with minimal disruption of the cellular material and surrounding tissue. It should be noted however that removal of some of the remnants of the tumour resulting from the ablation step may take place so long as sufficient tumour antigens (e.g. neo-antigens) remain to result in uptake and presentation by the autologous immature dendritic cells or precursor thereof, which are administered together with the checkpoint inhibitor in accordance with the invention, and an anti-tumour immune response.

Likewise, the ablative method employed is restricted only insofar as sufficient tumour antigens are generated to result in uptake and presentation thereof by the autologous immature dendritic cells or precursor thereof, which are administered together with the checkpoint inhibitor in accordance with the invention, and an anti-tumour immune response. Levels of tumour antigens may be monitored and measured by the means described above.

Tumour ablation methods are cryoablation, hydrothermal ablation, ionising radiation ablation (external beam radiation therapy or brachytherapy), radioablation, ultrasound ablation, laser ablation, microwave ablation and electroablation. Such methods are well practiced in the field of cancer therapy and so the skilled person would have no problem in performing such methods in line with accepted clinical practices.

Nonetheless, the inventors have found that cryoablation represents a particularly effective means to generate tumour antigens that are readily taken up and presented by the autologous immature dendritic cells which are administered together with the checkpoint inhibitor in accordance with the invention and which result in a, preferably robust, anti-tumour immune response.

A particular protocol for cryoablation of a tumour, specifically a prostate tumour, is provided in the Examples below. Thus, in certain embodiments the tumour undergoing treatment will be subjected to cryoablative temperatures at less than or equal to about -40°C (e.g. -45°C to -35°C, -42°C to -38°C, or -41 °C to - 39°C) followed by warming to about 37°C (e.g. 34°C to 39°C, 35°C to 38°C, or 36°C to 37°C) for a time sufficient to generate tumour antigens that are readily taken up and presented by the autologous immature dendritic cells which are administered together with the checkpoint inhibitor in accordance with the invention and which result in a robust anti-tumour immune response. Freezing and/or warming may conveniently be accomplished by the delivery of pressurized gas, e.g. argon, helium, neon, krypton and xenon gases. Argon may be especially convenient for cooling and helium may be especially convenient for warming. Preferably gases may be delivered through the same cryoprobe. A plurality or freeze/thaw cycles may be performed, e.g. at least 2, 3, 4 or 5. Two cycles may be advantageous.

In further embodiments cryoablation may be performed using the commercially available Endocare Cryocare CS™ system (Endocare, Inc.). The CS system also uses liquid argon gas as a cryogen, and liquid helium as a warming agent. Through thermocouple feedback, this system allows for controlled freezing of the volume of the tissue targeted, and "active" thawing of the same volume, either at the discretion of the physician or automatically via use of a computer-mediated system. In addition, the Cryocare CS™ system employs integrated ultrasound, which allows the operator to monitor all aspects of planning, probe placement and the progress of the freezing event via ultrasound on one unit.

In certain embodiments the methods and uses of the invention comprise a step prior to administration of the immune cell checkpoint inhibitor and the autologous immature DCs or precursor thereof in which the tumour is at least partially ablated, e.g. at least partially ablated with the ablation techniques recited herein, preferably cryoablation. Such methods have been shown by the inventors to be surprisingly well-tolerated by patients without any (or at least surprisingly limited levels of) surrounding tissue damage and associated infection, fistula formation and similar local anatomical deformations.

References to a tumour encompass any collection (mass) of neoplastic cells in intimate contact with one another. References to a neoplastic cell include any or all of malignant, pre-malignant and non-malignant (benign) neoplastic cells. The term tumour therefore encompasses, inter alia, neoplasms, cancers, malignancies, sarcoma, carcioma, germinoma, lymphona, leukaemia, blastoma, papilloma and adenoma insofar as these neoplasms are characterised by a collection (mass) of cells. Such collections or masses may be described as "solid" even though the masses may be diffuse and/or comprise voids. In certain embodiments the tumour is a malignant or premalignant tumour, e.g. a sarcoma, carcinoma, germinoma, blastoma, lymphoma or leukaemia.

In more specific embodiments the tumour may be a colorectal tumour (also known as colon tumour, rectal tumour or bowel tumour), prostate tumour, testicular tumour, skin tumour (e.g. melanoma and non-melanoma (e.g. basal-cell tumour, squamous-cell tumour)), breast tumour, kidney (renal) tumour (e.g. Wilm's tumour), ovarian tumour, stomach (gastric) tumour, intestinal tumour (e.g. duodenal tumour, ileal tumour, jejunal tumour, small intestine tumour), liver (hepatic) tumour, pancreatic tumour, lung (pulmonary) tumour, oesophageal tumour, oral tumour, throat tumour, brain tumour (e.g. glioblastoma, medulloblastoma), adrenal tumour (e.g. adrenocortical tumour), thyroid tumour (e.g. anaplastic thyroid carcinoma), uterine tumour (e.g. uterine carcinosarcoma), haematological tumour (also known as the haematological malignancies) (e.g. haematopoietic and lymphoid tumour malignancies, e.g. leukaemia, lymphoma and myeloma).

In more specific embodiments the tumour may be a colorectal cancer (also known as colon cancer, rectal cancer or bowel cancer), prostate cancer, testicular cancer, skin cancer (e.g. melanoma and non-melanoma (e.g. basal-cell cancer, squamous-cell cancer)), breast cancer, kidney (renal) cancer (e.g. Wilm's tumour), ovarian cancer, stomach (gastric) cancer, intestinal cancer (e.g. duodenal cancer, ileal cancer, jejunal cancer, small intestine cancer), liver (hepatic) cancer, pancreatic cancer, lung (pulmonary) cancer, oesophageal cancer, oral cancer, throat cancer, brain cancer (e.g. glioblastoma, medulloblastoma), adrenal cancer (e.g. adrenocortical cancer), thyroid cancer (e.g. anaplastic thyroid carcinoma), uterine cancer (e.g. uterine carcinosarcoma), haematological cancer (also known as the haematological malignancies) (e.g. haematopoietic and lymphoid cancer malignancies, e.g. leukaemia, lymphoma and myeloma) or a non-malignant tumour in these anatomical sites (e.g. colorectal polyps, pilomatrixoma, hemangioma, osteoma, chondroma, lipoma, fibroma, lymphangioma, leiomyoma, rhabdomyoma, astrocytoma, meningioma, ganglioneuroma, papilloma, adenoma).

The present invention may find particular application in metastatic cancers (e.g. metastatic forms of the above listed cancers) because the anti-tumour immune responses induced by the methods of the invention will be directed not only to the primary ablated tumour, but also any metastases (secondary tumours) thereof which have established or may be becoming established elsewhere in the subject's body, e.g. in bone and soft tissues including the liver, lymph nodes, brain, lung. The treatment of metastatic castration resistant prostate cancer (mCRCP) is of particular note.

In other embodiments the methods of the invention may further comprise a following step in which the subject's clinical indicators of the tumour and/or the treatment, are assessed and preferably compared to a corresponding assessment made prior to, or earlier in, said treatment in order to determine any changes therein. Disease progression and treatment effects may be assessed by *in vivo* radiology and nuclear medicine imaging of the tumour. Suitable techniques include, but are not limited to, magnetic resonance imaging (MRI), contrast enhanced computer tomography (diagnostic CT), single photon emission computer tomographic (SPECT) and positron emission tomography (PET).

Disease progression and treatment effects may alternatively or additionally be assessed by molecular analysis of the subject's immune response, e.g. monitoring and measuring levels of various immune cell population and various cytokines in the subject's blood or other tissues and body fluids. Many techniques may be employed to effect this analysis but a theme common to many is the use of antibodies which bind specifically to said cytokines and immune cell markers indicative of immune cell types. The binding of these antibodies to their target may be detected and/or quantified by flow cytometry based techniques or immunochemical assays. Convenient cell markers include but are not limited to, CD4, CD8, CD11b, CD14, CD15, CD25, CD33, CD39, CD124, CD127, HLA-DR, FOXP3, MHC tetramer staining. Cytokines which may be monitored include, but are not limited to, IFN-γ, IL-1β, IL-2, IL-, IL-6, IL-8, IL-10, IL-12p70, IL-13 and TNFα. The levels of tumour specific antibodies in a subject's blood or other tissues and body fluids can also be detected and quantified with panels of commonly seen antigens and appropriate immunochemical techniques.

Disease progression and treatment effects may alternatively or additionally be assessed by detecting, and optionally measuring the levels of, circulating tumour cells, circulating free tumour DNA and/or blood mRNA. Suitable techniques are available in the art, e.g. Yu M., et al. 2011, Journal of Cell Biology, 192(3), 373-382; Bettegowda, C., et al., 2014, Science Translational Medicine, 6(224), 224ra24; and Ross, R.W., et al., 2012, The Lancet Oncology, 13(11), 1114-1124.

Disease progression and treatment effects may alternatively or additionally be assessed by detecting, and optionally measuring the levels, e.g. circulating levels, of cancer biomarkers. For instance PSA, PSMA, PAP may be used as biomarkers in the case of prostate cancer; carcinoembryonic antigen (CEA) levels may be indicative of colorectal, gastric, pancreatic, lung, breast, and thyroid cancer; and levels of CA-125 may be indicative of ovarian and uterine cancer.

The subject may be any human or non-human animal subject, but more particularly may be a human or non-human vertebrate, e.g. a non-human animal selected from mammals, birds, amphibians, fish and reptiles. The non-human animal may be a livestock or a domestic animal or an animal of commercial value, including laboratory animals or an animal in a zoo or game park. Representative non-human animals therefore include dogs, cats, rabbits, mice, guinea pigs, hamsters, horses, pigs, sheep, goats, cows, chickens, turkeys, guinea fowl, ducks, geese, parrots, budgerigars, pigeons, salmon, trout, tilapia, catfish, bream, barramundi, grouper, mullet, amberjack, croaker, rohu, goby, cod, haddock, sea bass and carp. Veterinary uses of the invention are thus covered. The subject may be viewed as a patient. Preferably the subject is a human.

"Treatment" when used in relation to the treatment of a tumour in a subject in accordance with the invention is used broadly herein to include any therapeutic effect, i.e. any beneficial effect on, or in relation to, the tumour. Thus, not only included is eradication or elimination of the tumour, or cure of the subject of the tumour, but also an improvement in the general condition and/or well-being of the subject or a specific condition associated with the tumour or a halt to a deterioration in the general condition or well-being of the subject or a specific condition associated with the tumour. Thus included, for example, is an improvement in any symptom or sign of the tumour, or in any clinically accepted indicator of the tumour (for example a decrease in tumour size (volume, area and/or cell number), a decrease in tumour invasion, a decrease in the number of circulating tumour cells, or a reduction in general discomfort or pain in the surrounding tissue and systemically). Treatment thus includes both curative and palliative therapy, e.g. of a pre-existing or diagnosed tumour, i.e. a reactionary treatment.

Treatment also includes preventative effects on the tumour and associated conditions. It thus includes delaying, limiting, reducing or preventing a condition associated with the tumour, or the onset of a condition associated with the tumour, or one or more symptoms or indications thereof (e.g. an increase in the size of a tumour or the development of secondary tumours or an increase in the number of circulating tumour cells), for example relative to the condition or symptom or indication prior to the treatment. Prevention thus explicitly includes both absolute prevention of occurrence or development of a condition associated with the tumour, or symptom or indication thereof, and any delay in the onset or development of a condition associated with the tumour or symptom or indication, or reduction or limitation on the development or progression of the condition associated with the tumour or symptom or indication thereof.

Treatment therefore further includes stabilising a condition or symptom associated with the tumour or stabilising the general condition or well-being of the subject undergoing treatment (i.e. preventing or reducing the worsening of a condition or symptom associated with the tumour or the general condition or well-being of the subject).

It will be apparent that in the context of the treatment of tumours and cancer (especially late stage tumours and cancers), a treatment may further be considered effective if the life expectancy of the subject is extended, even slightly, in particular if such extended life is provided whilst providing a meaningful quality of life. Moreover a treatment may be considered effective if quality of life is enhanced even if life expectancy is not improved.

As shown in the Examples, the treatments of the invention are preferably associated with fewer adverse side effects, e.g. autoimmune adverse events (including autoimmune colitis, hepatitis, uveitis, hypophysitis and autoimmune skin disorders). Such adverse effects are typically seen with the use of immune cell check point inhibitors in the treatment of cancer. As also shown in the Examples, the treatments of the invention are preferably associated with low levels of surgical side effects, including caused by surrounding tissue damage, e.g. infection, fistula formation and similar local anatomical deformations.

In particular embodiments the treatments of the invention at least stabilise a condition or symptom associated with the tumour or at least stabilise the general condition or well-being of the subject undergoing treatment (i.e. at least prevent or reduce the worsening of a condition or symptom associated with the tumour or the general condition or well-being of the subject) or increase life expectancy of the subject undergoing treatment without eliciting serious adverse side effects, e.g. autoimmune colitis, hepatitis, uveitis, hypophysitis and autoimmune skin disorders.

In particular embodiments the treatments of the invention at least stabilise a condition or symptom associated with the tumour (i.e. at least prevent or reduce the worsening of a condition or symptom associated with the tumour) or increase life expectancy of the subject undergoing treatment without reducing, in particular enhancing, quality of life.

In the therapeutic methods of the invention the DCs or precursor thereof and the immune cell checkpoint inhibitor of use in the invention will be administered (applied) to the subject in "pharmaceutically effective" or "physiologically effective" amounts. A "pharmaceutically/physiologically effective" amount of the immune cell checkpoint inhibitor or DCs or precursor thereof of use in the invention is that amount of inhibitor and/or DC which provides measurable treatment of the target tumour as described herein.

In connection with the immune cell checkpoint inhibitors of use in accordance with the invention, this may also be expressed as a therapeutically effective reduction in the activities of TReg cells or augmentation of the activity of T_{C} cells on the immune response induced by the autologous immature dendritic cells which are administered together with the checkpoint inhibitor in accordance with the invention.

In connection with DCs or precursors thereof of use in accordance with the invention, this may also be expressed as a therapeutically effective induction or enhancement of the immune response against the tumour.

A "pharmaceutically effective" or "physiologically effective" amount can be determined with reference to standard practices for deciding dosage amounts and the skilled person will be able to detect evidence of successful treatment from his experience and with the aid of routine tests available to him that are designed to monitor the targeted tumour and associated conditions, and those which are designed to monitor the effects of the DCs or precursors thereof and the effects of the immune cell checkpoint inhibitors, e.g. those tests described herein, in particular on the immune response of the subject against the tumour and on the activities of TReg and T_{C} cells in the subject.

In other embodiments a further pharmaceutical useful in the treatment of neoplastic disease, in particular the immunotherapeutic treatment of neoplastic disease, is administered together with the immune cell checkpoint inhibitor and the autologous immature DCs or precursor thereof. The term "together with" in these embodiments should be construed in accordance with the above use of the term.

The further pharmaceutical (i.e. further therapeutically active agent) may be a cytotoxic chemotherapy agent, an anti-hormonal agent (agents that act to regulate or inhibit hormone action on tumours), an angiogenesis inhibitor, an anti-cancer monoclonal antibody, a radioimmunotherapeutic, a cancer treatment vaccine, an immunostimulatory agent or an immunosuppressant.

Representative examples of suitable cytotoxic chemotherapy agents include, but are not limited to, alkylating agents; e.g. thiotepa and cyclophosphamide; alkyl sulfonates, e.g. busulfan, improsulfan and piposulfan; aziridines, e.g. benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards, e.g. chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas, e.g. carmustine, chlorozotocin, fotemustine, lomustine, nimustine, ranimustine; antibiotics, e.g. aclacinomysins, actinomycin, authramycin, azaserine, bleomycin, cactinomycin, calicheamicin, carabicin, carminomycin, carzinophilin, chromomycin, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites, e.g. methotrexate and 5-fluorouracil (5-FU); folic acid analogues, e.g. denopterin, methotrexate, pteropterin, trimetrexate; purine analogues, e.g. fludarabine, 6-mercaptopurine, thiamiprine, thioguanine; pyrimidine analogues, e.g. ancitabine, azacitidine, 6-azauridine, carmofur, cytarabine, dideoxyuridine, doxifluridine, enocitabine, floxuridine, 5-FU; androgens, e.g. calusterone, dromostanolone propionate, epitiostanol, mepitiostane, testolactone; anti-adrenals, e.g. aminoglutethimide, mitotane, trilostane; folic acid vitamer, e.g. folinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK(R); razoxane; sizofiran; spirogermanium; tenuazonic acid; triaziquone; 2, 2', 2" - trichlorotriethylamine; urethan; vindesine; dacarbazine; mannomustine; mitobronitol; mitolactol; pipobroman; gacytosine; arabinoside; thiotepa; taxanes, e.g. paclitaxel and docetaxel; chlorambucil; gemcitabine; 6-thioguanine; mercaptopurine; methotrexate; platinum analogues, e.g. cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; esperamicins; and capecitabine.

Representative examples of suitable anti-hormonal agents include, but are not limited to, anti-estrogens e.g. tamoxifen, raloxifene, aromatase inhibiting 4(5)-imidazoles, 4-hydroxytamoxifen, trioxifene, keoxifene, LY 117018, onapristone, and toremifene(Fareston); and anti-androgens, e.g. flutamide, nilutamide, bicalutamide, leuprolide, and goserelin.

Representative examples of suitable angiogenesis inhibitors include, but are not limited to, bevacizumab, everolimus, lenalidomide, pazopanib, ramucirumab sorafenib, sunitinib and thalidomide.

Representative examples of suitable anti-cancer monoclonal antibody include, but are not limited to, alemtuzumab, bevacizumab, cetuximab, ipilimumab, nivolumab, ofatumumab, panitumumab, pembrolizumab, rituximab, and trastuzumab.

Representative examples of suitable radioimmunotherapeutics include, but are not limited to, ibritumomab and tositumomab.

Representative examples of suitable cancer treatment vaccines include, but are not limited to, sipuleucel-T.

Representative examples of immunostimulatory agents include, but are not limited to, cytokines e.g. TNF, IFNα, IL-1, IL-2 and inhibitors of inhibitory immune checkpoints, e.g. ipilimumab, nivolumab and pembrolizumab and any others described herein.

Representative examples of immunosuppressants include, but are not limited to, cyclosporine, rapamycin, tacrolimus, dactinomycin, mitomycin c, bleomycin, mithramycin, azathioprine, hydrocortisone, cortisone, prednisone, prednisolone, methylprednisolone, bexamethasone, betamethasone, triamcinolone, beclomethasone, fludrocortisone acetate, deoxycorticosterone acetate and aldosterone.

The use of the above further therapeutic agents, in particular the cytotoxic chemotherapy agents, in a metronomic (low) dosage regime (long-term exposure to frequent and (usually) relatively low doses with no drug-free breaks) may be advantageous. Such metronomic dosage regimes may begin before during or after the administration of the immune cell checkpoint inhibitor and the autologous immature DCs or precursor thereof and last at least 1 month, e.g. at least 2, 3, 4, 5, 6, 8, 10 or 12 months. If the metronomic dosage regime begins after the administration of the immune cell checkpoint inhibitor and the autologous immature DCs or precursor thereof, this may be no later than 30 days, e.g. no later than 25, 20, 15, 12, 10, 8, 7, 6, 5, 3, 2 or 1 days, after said administration.

Of particular note is the use of cyclophosphamide (Cy) together with the immune cell checkpoint inhibitor and the autologous immature DCs or precursors thereof. Without wishing to be bound by theory, it is believed that administration of Cy at the time of administering antitumour vaccines will combat the increase in TReg cell population seen following cancer vaccination which may dampen the immune response to the vaccine (Zhou G, et al., 2006, Blood 107, 628-636). This is because TReg populations have been shown to be sensitive Cy, in particular at sub-clinical doses (in the sense of its action as a chemotherapeutic) where circulating TReg have been shown to diminish with very little impact on other white cell populations (Bass and Mastrangelo, 1998; Cancer immunology, immunotherapy: CII 47, 1-12; and Ghiringhelli et al, 2004, European Journal of Immunology 34:336-344). In this regard Cy has been used as an immunopotentiating agent in a number of clinical trials of the active-specific immunotherapy (e.g. cancer vaccine) of advanced cancer. In these studies low-dose (typically 300 mg/m²) Cy was administered intravenously 3-4 days prior to the vaccine regimen (Bass and Mastrangelo, 1998). Mild to no toxicity was noted across eleven trials involving 285 subjects at the 300 mg/m2 dose of Cy (Bass and Mastrangelo, 1998).

Thus, in certain embodiments of the methods and uses of the invention Cy (which term includes prodrugs or metabolites thereof (e.g. 4-hydroxy cyclophosphamide and N,N-bis(2-chloroethyl)phosphorodiamidic acid)) is a administered together with the immune cell checkpoint inhibitor and the autologous immature DCs or precursor thereof. The term "together with" in these embodiments should be construed in accordance with the above use of the term.

In certain embodiments Cy is administered repeatedly, e.g. at least once prior to the administration of the immune cell checkpoint inhibitor and the autologous immature DCs or precursor thereof, e.g. no more than 10 days prior or no more than 8, 6, 5 4, 3, 2 or 1 days prior. Preferably Cy is administered at least once about 3 days prior, e.g 3.5 to 2.5 days prior to the administration of the immune cell checkpoint inhibitor and the autologous immature DCs or precursor thereof.

In further embodiments Cy is administered at least once subsequent to an administration of the immune cell checkpoint inhibitor and the autologous immature DCs or precursor thereof (which administration may be the first, the final or any intervening administration). In these embodiments it may be advantageous to administer Cy in a cyclical pattern, i.e. a timeperiod in which therapeutic levels of Cy are maintained in the subject and a timeperiod in which the subject does not receive Cy. This may be a plurality of days (e.g. 2, 3, 4, 5, 6, 7, 8, 9 or 10 days) receiving Cy followed by a plurality of days not receiving Cy (e.g. 2, 3, 4, 5, 6, 7, 8, 9 or 10 days). The number of cycles is not restricted and may be continued so long as a beneficial effect on the anti-tumour immune response is observed. For instance it may be advantageous for the subject to receive 1, 2, 3, 4, 5, 6, 8, 10, 12, 14, 16, 18 or 20 cycles of about 7 days receiving Cy and about 7 days of not receiving Cy. In these embodiments it may alternatively be advantageous to administer Cy in a metronomic dosage regime, e.g. those described above. A metronomic dosage regime beginning about 7 days after the administration of the immune cell checkpoint inhibitor and the autologous immature DCs or precursor thereof and lasting for at least 6 months may be advantageous. Suitable doses of Cy may be determined by the skilled person without undue burden based on the subject's physical characteristics and the characteristics of the tumour and/or by monitoring circulating levels of TReg cells (i.e. CD4+ CD25+ Foxp3+ CLTA4+ and GTIR+ T cel, or more generally CD4+ and CD25+ T cells) in the subject's blood, as described extensively in the art (e.g. in Ghiringhelli et al., 2007, Cancer Immunology, Immunotherapy 56:641-648). Thus, a suitable dose of Cy will be that which reduces the circulating levels of TReg cells in a subject's blood as compared to the levels of said cells prior to Cy treatment. In practice this may be that dose which maintains the levels of said TReg cells at the levels seen prior to administration of the autologous immature DCs or precursors thereof or which at least prevents a significant rise in the circulating levels of said TReg cells following administration of administration of the autologous immature DCs or precursors thereof. In preferred embodiments a dose is selected which does not also significantly lower circulating levels of leukocytes, and lymphocytes in particular, other than TReg cells.

Suitable doses for iv administration may be 1 to 500 mg/m², e.g. 10 to 500 mg/m², 50 to 500 mg/m², 100 to 500 mg/m², 150 to 500 mg/m², 200 to 500 mg/m², 250 to 500 mg/m², 300 to 500 mg/m², 350 to 500 mg/m², 400 to 500 mg/m², 450 to 500 mg/m², 1 to 450 mg/m², 1 to 400 mg/m², 1 to 300 mg/m², 1 to 250 mg/m², 1 to 200 mg/m², 1 to 150 mg/m², 1 to 100 mg/m², 1 to 50 mg/m², or 1 to 10 mg/m². Doses of about 300 mg/m², e.g. 250 to 350 mg/m², 275 to 325 mg/m², or 290 to 310 mg/m², may be advantageous. Any ranges which may be formed from the range endpoints recited above are explicitly contemplated.

Suitable daily doses for oral administration may be 1 to 100 mg, e.g. 5 to 100 mg, 10 to 100 mg, 20 to 100 mg, 25 to 100 mg, 30 to 100 mg, 35 to 100 mg, 40 to 100 mg, 45 to 100 mg, 50 to 100 mg, 55 to 100 mg, 60 to 100 mg, 65 to 100 mg, 70 to 100 mg, 75 to 100 mg, 80 to 100 mg, 90 to 100 mg, 1 to 100 mg, 1 to 90 mg, 1 to 80 mg, 1 to 75 mg, 1 to 70 mg, 1 to 65 mg, 1 to 60 mg, 1 to 55 mg, 1 to 50 mg, 1 to 45 mg, 1 to 40 mg, 1 to 35 mg, 1 to 30 mg, 1 to 25 mg, 1 to 20 mg, 1 to 10 mg, or 1 to 5 mg. Daily oral doses of about 50 mg, e.g. 30 to 70 mg, 40 to 60 mg, or 45 to 55 mg, may be advantageous. Daily oral doses may be given in a plurality of administrations, e.g. twice daily (b.i.d) or three times daily. The amount administered each time will be adjusted accordingly for the frequency selected. Any ranges which may be formed from the range endpoints recited above are explicitly contemplated.

In accordance with the invention Cy and prodrugs or metabolites thereof (e.g. 4-hydroxy cyclophosphamide and N,N-bis(2-chloroethyl)phosphorodiamidic acid) is not an immune cell checkpoint inhibitor as defined herein.

The skilled man will be able to formulate the immune cell checkpoint inhibitor of use in the invention as herein defined into pharmaceutical compositions that are adapted for local administration to the site of the ablated tumour or part thereof according to any of the conventional methods known in the art and widely described in the literature. Such compositions may, at their simplest, be a solution of the inhibitor in sterile water.

The present invention therefore also provides a pharmaceutical composition for use in any of the above-mentioned methods or uses comprising an immune cell checkpoint inhibitor of use in the invention as herein defined, together with at least one pharmaceutically acceptable carrier, diluent or excipient, preferably in an amount sufficient to combat the activities of TReg cells or augment the activity of T_{C} cells on the immune response induced by the autologous immature dendritic cells which are administered together with the checkpoint inhibitor in accordance with the invention. The composition may comprise a plurality of immune cell checkpoint inhibitors. The composition may also comprise other therapeutic agents, e.g. those described above. The composition may also comprise an autologous immature DC or precursor thereof as described herein.

More specifically, the immune cell checkpoint inhibitors of use in the invention as herein defined may be incorporated, optionally together with one another active agents, with one or more conventional carriers, diluents and/or excipients, to produce conventional galenic preparations for localised delivery to the site of the ablated tumour or part thereof, e.g. implantable tablets, pills, lozenges, and soft and hard gelatine capsules, powders, suspensions, emulsions, solutions, aerosols (as a solid or in a liquid medium), sprays (e.g. nasal sprays), compositions for use in nebulisers, ointments, creams, salves, suppositories, pessaries, sterile injectable solutions, sterile packaged powders, and the like. Sterile injectable compositions are of particular note.

Examples of suitable carriers, excipients, and diluents are lactose, dextrose, sucrose, sorbitol, mannitol, starches, gum acacia, calcium phosphate, inert alginate polymers, tragacanth, gelatine, calcium silicate, microcrystalline cellulose, polyvinylpyrrolidone, cellulose, water syrup, water, water/ethanol, water/ glycol, water/polyethylene, hypertonic salt water, glycol, propylene glycol, methyl cellulose, methylhydroxybenzoates, propyl hydroxybenzoates, talc, magnesium stearate, mineral oil or fatty substances, e.g. hard fat or suitable mixtures thereof. Excipients and diluents of note are mannitol and hypertonic salt water (saline).

The compositions may additionally include lubricating agents, wetting agents, emulsifying agents, suspending agents, preserving agents, sweetening agents, stabilising agents, e.g. buffers and antioxidants, flavouring agents, and the like. Additional therapeutically active agents may be included in the pharmaceutical compositions if required.

Parenterally administrable forms, e.g. solutions suitable for localised delivery to the site of the ablated tumour or part thereof via intravenous, intraarterial, intraosseous, intra-muscular, intracerebral, intrathecal, subcutaneous, intradermal, intrapancreatic, intratumoural routes or via the tumour vasculature, e.g. by injection, infusion, or perfusion, should be sterile and free from physiologically unacceptable agents, and should have low osmolarity to minimize irritation or other adverse effects upon administration. Thus such solutions should preferably be isotonic or slightly hypertonic, e.g. hypertonic salt water (saline). Suitable vehicles include aqueous vehicles customarily used for administering parenteral solutions, e.g. sterile water for injection, Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975)), which is referred to herein. The solutions can contain preservatives, antimicrobial agents, buffers and antioxidants conventionally used for parenteral solutions, excipients and other additives which are compatible with the biopolymers and which will not interfere with the manufacture, storage or use of products.

Simple sterile solutions of immune cell checkpoint inhibitors of use in the invention as herein defined or simple sterile liquid compositions comprising immune cell checkpoint inhibitors of use in the invention as herein defined may be especially convenient for use during surgical procedures and for delivery to the airways, e.g. by nebuliser or nasal spray device.

For topical administration the immune cell checkpoint inhibitors of use in the invention as herein defined can be incorporated into creams, ointments, gels, salves, transdermal patches and the like. Further topical systems that are envisaged to be suitable are implantable 3D scaffolds, for example sponges and gels, e.g. solid, semi-solid, amorphous or liquid crystalline gel matrices. Such systems may be biodegradable. Such scaffolds can conveniently be designed to control the release of the inhibitor from the matrix, e.g. release can be delayed and/or sustained over a chosen period of time.

In certain embodiments such 3D scaffold systems, in particular gel-based systems, may be formed in situ upon contact with biological tissues or fluids, e.g. mucosal surfaces. Typically such gels are bioadhesive and/or mucoadhesive. Delivery to any body site that can retain or be adapted to retain the pre-gel composition can be targeted by such a delivery technique, e.g. a tumour or the remnants of a tumour and/or surrounding tissue following ablation or other such destruction. Such systems are described in WO 2005/023176, which is referred to herein.

The compositions and products containing an immune cell checkpoint inhibitor of use in the invention will typically comprise 1% to 99%, 2% to 98%, 5% to 95%, 10% to 90%, 15% to 85% or 25% to 75% w/w or w/v (as appropriate) of the immune cell checkpoint inhibitors of use in the invention as herein defined, allowance being made for other ingredients, e.g. further therapeutic agents and pharmaceutically acceptable excipients.

The precise content of the immune cell checkpoint inhibitors of use in the invention as herein defined in the compositions and products of the invention can vary depending on the dosage required and the dosage regime being followed but will be in an amount sufficient to combat the activities of TReg cells or augment the activity of T_{C} cells on the immune response induced by the autologous immature dendritic cells which are administered together with the checkpoint inhibitor in accordance with the invention, taking account of variables such as the physical size of the subject to be treated, the nature of the subject's particular ailments, and the location and identity of the target treatment area. The skilled man would know that the amounts of inhibitor can be reduced if a multiple dosing regimen is followed or increased to minimise the number of administrations or applications.

A representative aqueous solution for parenteral delivery, e.g. those routes recited above, will be sterile and may contain 1 to 25%, 1 to 20%, 1 to 15%, 1 to 10%, 1 to 9%, 1 to 8%, 1 to 7%, 1 to 6%, 1 to 5%, 1 to 2%, 2 to 25%, 2 to 20%, 2 to 15%, 2 to 10%, 2 to 9%, 2 to 8%, 2 to 7%, 2 to 6%, 2 to 5%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the inhibitor, the remainder being comprised of water and pharmaceutically acceptable excipients, and other active agents and/or autologous immature DCs or a precursor thereof if being used.

A representative topical formulation, e.g. a cream, ointment or salve, which may be used to administer an immune cell checkpoint inhibitor of use in the invention as herein defined to the skin might contain 1 to 25%, 1 to 20%, 1 to 15%, 1 to 10%, 1 to 9%, 1 to 8%, 1 to 7%, 1 to 6%, 1 to 5%, 1 to 2%, 2 to 25%, 2 to 20%, 2 to 15%, 2 to 10%, 2 to 9%, 2 to 8%, 2 to 7%, 2 to 6%, 2 to 5%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the inhibitor, the remainder being comprised of pharmaceutically acceptable excipients, and other active agents and/or autologous immature DCs or a precursor thereof if being used.

The skilled man will be able to formulate the autologous immature DCs or a precursor thereof of use in the invention as herein defined into pharmaceutical compositions that are adapted for local administration to the site of the ablated tumour or part thereof according to any of the conventional methods known in the art and widely described in the literature. Such compositions may, at their simplest, be a suspension of the cells in sterile water. In other common embodiments such compositions may be a suspension of cells in a buffered aqueous solution, a cell culture medium or a cryopreservation medium.

The present invention therefore also provides a composition for use in any of the above-mentioned methods or uses comprising an autologous immature DCs or a precursor thereof of use in the invention as herein defined, together with at least one pharmaceutically acceptable carrier, diluent or excipient, preferably in an amount sufficient to uptake and present tumour antigens derived from the at least partially ablated tumour and induce in an immune response directed against said antigens. The composition may also comprise other therapeutic agents, e.g. those described above. The composition may also comprise an immune cell checkpoint inhibitor as described herein. The compositions may additionally include stabilising agents, e.g. buffers and antioxidants, suspending agents, preserving agents, e.g. antimicrobial agents, cryopreservation agents and the like.

Examples of suitable carriers, excipients, and diluents are lactose, dextrose, sucrose, sorbitol, mannitol, starches, gum acacia, calcium phosphate, inert alginate polymers, tragacanth, gelatine, calcium silicate, microcrystalline cellulose, polyvinylpyrrolidone, cellulose, water syrup, water, water/ethanol, water/ glycol, water/polyethylene, hypertonic salt water, glycol, propylene glycol, methyl cellulose, methylhydroxybenzoates, propyl hydroxybenzoates, talc, magnesium stearate, mineral oil or fatty substances, e.g. hard fat or suitable mixtures thereof. Sugars, salts and essential amino acids which may be metabolised by the cells may also be included

Parenterally administrable forms, e.g. solutions suitable for localised delivery to the site of the ablated tumour or part thereof via intravenous, intraarterial, intraosseous, intra-muscular, intracerebral, intrathecal, subcutaneous, intradermal, intrapancreatic, intratumoural routes or via the tumour vasculature, e.g. by injection, infusion, or perfusion, should be sterile and free from physiologically unacceptable agents, and should have low osmolarity to minimize irritation or other adverse effects upon administration. Thus such solutions should preferably be isotonic or slightly hypertonic, e.g. hypertonic salt water (saline). Suitable vehicles include aqueous vehicles customarily used for administering parenteral solutions, e.g. sterile water for injection, Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975)), which is referred to herein.

For topical administration the autologous immature DCs or a precursor thereof of use in the invention as herein defined can be incorporated into creams, ointments, gels, salves, transdermal patches and the like. Further topical systems that are envisaged to be suitable are implantable 3D scaffolds, for example sponges and gels, e.g. solid, semi-solid, amorphous or liquid crystalline gel matrices, which carry the cells and release the cells over time to the site of the ablated tumour. Such systems may be biodegradable.

In certain embodiments such 3D scaffold systems, in particular gel-based systems, may be formed in situ upon contact with biological tissues or fluids, e.g. mucosal surfaces. Typically such gels are bioadhesive and/or mucoadhesive. Delivery to any body site that can retain or be adapted to retain the pre-gel composition can be targeted by such a delivery technique, e.g. a tumour or the remnants of a tumour and/or surrounding tissue following ablation or other such destruction. Such systems are described in WO 2005/023176, which is referred to herein.

The compositions and products containing the autologous immature DCs or a precursor thereof of use in the invention will typically comprise 1% to 99%, 2% to 98%, 5% to 95%, 10% to 90%, 15% to 85% or 25% to 75% w/w or w/v (as appropriate) of the autologous immature DCs or a precursor thereof of use in the invention as herein defined, allowance being made for other ingredients, e.g. further therapeutic agents and pharmaceutically acceptable excipients.

A representative aqueous solution for parenteral delivery, e.g. those routes recited above, should be sterile and may contain 1 to 25%, 1 to 20%, 1 to 15%, 1 to 10%, 1 to 9%, 1 to 8%, 1 to 7%, 1 to 6%, 1 to 5%, 1 to 2%, 2 to 25%, 2 to 20%, 2 to 15%, 2 to 10%, 2 to 9%, 2 to 8%, 2 to 7%, 2 to 6%, 2 to 5%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the autologous immature DCs or a precursor thereof, the remainder being comprised of water and pharmaceutically acceptable excipients, and other active agents and/or an immune checkpoint inhibitor if being used.

A representative 3D scaffold which may be used to administer the autologous immature DCs or a precursor thereof of use in the invention as herein defined to an internal site of tumour ablation might contain 1 to 25%, 1 to 20%, 1 to 15%, 1 to 10%, 1 to 9%, 1 to 8%, 1 to 7%, 1 to 6%, 1 to 5%, 1 to 2%, 2 to 25%, 2 to 20%, 2 to 15%, 2 to 10%, 2 to 9%, 2 to 8%, 2 to 7%, 2 to 6%, 2 to 5%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the autologous immature DCs or a precursor thereof, the remainder being comprised of scaffolding materials, further pharmaceutically acceptable excipients, and other active agents and/or an immune checkpoint inhibitor if being used.

A representative topical formulation, e.g. a cream, ointment or salve which may be used to administer the autologous immature DCs or a precursor thereof of use in the invention as herein defined to the skin might contain 1 to 25%, 1 to 20%, 1 to 15%, 1 to 10%, 1 to 9%, 1 to 8%, 1 to 7%, 1 to 6%, 1 to 5%, 1 to 2%, 2 to 25%, 2 to 20%, 2 to 15%, 2 to 10%, 2 to 9%, 2 to 8%, 2 to 7%, 2 to 6%, 2 to 5%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the autologous immature DCs or a precursor thereof, the remainder being comprised of pharmaceutically acceptable excipients, and other active agents and/or an immune checkpoint inhibitor if being used.

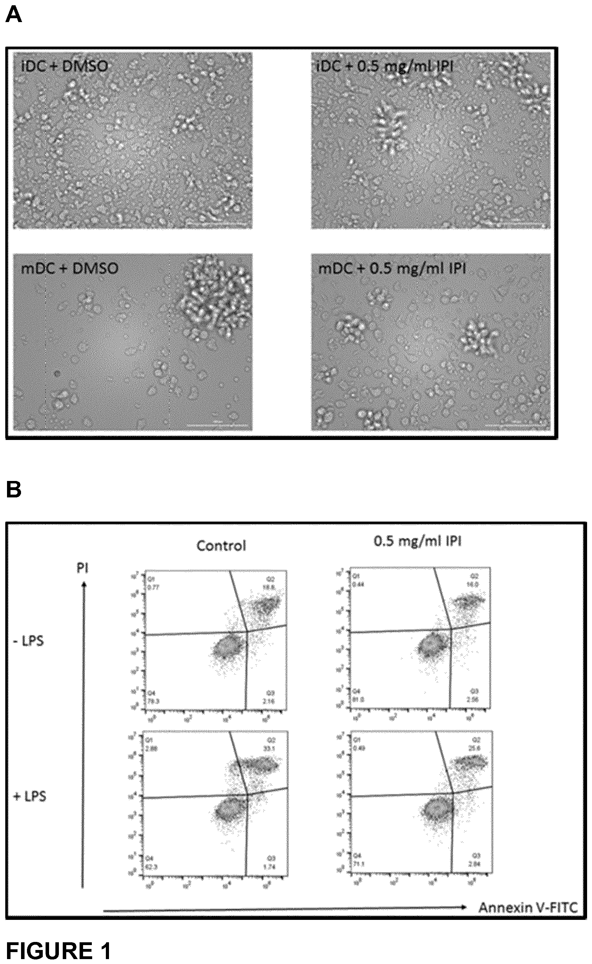

The invention will be further described with reference to the following nonlimiting Examples, in which
Figure 1 shows microscopic (A) and flow cytometric (B) analysis of DC cytotoxicity in the presence of 0.5 mg/ml ipilimumab or DMSO control for 24 hours.

### EXAMPLES

### Example 1 - Dendritic cell production

Following completion of leukapheresis of the patient's whole blood, the resultant cell product will contain an estimated 5 x 10¹⁰ leukocytes in an estimated volume of 400ml plasma. The leukapheresis product will be further enriched for monocyte content by processing on the ELUTRA® Cell Separation System following established and certified routines. ELUTRA® processing will result in the separation of the lymphocyte fraction from the mononuclear cell collection obtained by leukapheresis. The post-ELUTRA® product will thus be comprised predominantly of a CD14+ monocytic cell population, which will serve as the precursor of autologous induced immature dendritic cells (DCs).

DCs will then be differentiated from peripheral blood CD14+ monocytes collected during leukapheresis in a four-day ex vivo closed culture system in the presence of two cytokines: Granulocyte-Macrophage Colony Stimulating Factor (GM-CSF at 50ng/ml and IL4 at 20ng/ml).

Following three days of culture, the cells will be harvested, washed, and cryo-preserved in vials at a concentration of 5 x 10⁷ total cells/vial suspended in 1.0 ml of human-injectable (USP) cryopreservative media. A number of the vials will be thawed and the cells subjected to a panel of release testing regiments including identity and viability assays, a 14 day USP sterility test, mycoplasma test and endotoxin test. Immunophenotyping will then be used to assess the contents of induced immature dendritic cells, including markers CD14, CD80, CD83, CD86, CD40, CCR7, HLA-DR, CD3, CD19, CD16+CD56, CD66b. Induced immature dendritic cells are characterized by absence of CD14, not more than low expression of CD80, CD83, CD86, low expression of CD40, HLA-DR and no expression of CD3, CD19, CD16+CD56, CD66b.

Upon meeting the criteria for release, cryovials containing preserved autologous immature DCs will be stored in the gaseous phase of a dedicated liquid nitrogen tank at the Clinical Trial Unit until the patient is ready for intratumoural administration in combination with the cryotherapy procedure. This administration is described in Examples 2 and 3.

### Example 2 - Cryoablation of the tumour

Cryoablation of prostate tumours is currently an accepted, definitive treatment for primary prostate cancer (Long et al., 2001, Urology 57:518-523.). All subjects will be administered an intravenous dose of ciprofloxacin (in D5W (5% dextrose in water), 400 mg/200 ml) beginning one hour prior to the cryoablation procedure. Upon discharge, all subjects will continue ciprofloxacin therapy, 500 mg b.i.d. per orum for a period of eight days. Cryoablative temperatures, which have been histologically demonstrated to be -60°C when a single freeze is employed, and -40°C when a double freeze/thaw technique is used (Larson et al., 2000, Urology 55:547- 552).

Subjects will be treated under spinal or general anesthesia (according to subject preference) and sedation. Dependent upon the volume of the prostate gland to be treated, between six and eight sharpened 2.4 mm x 15 cm insulated cryoprobes (Endocare, Inc., Irvine, CA) will be placed into the prostate under transrectal ultrasound guidance (Bahn et al., 2002, Urology 60:3-11). Placement of the probes by the operator will be such that the three-dimensional "ice-ball" formed by the confluence of the contributing cryoprobes will contain the prostate tumour, but not extend to adjacent anatomy. Seminal vesicles will not be treated.

The prostate will then be subjected to cryoablative temperatures at less than or equal to -40°C followed by warming to 37°C. Freezing and warming will be accomplished by the delivery of pressurized argon and helium gases, respectively, through the same cryoprobe. At the time of the placement of the cryoprobes into the prostate under ultrasound guidance, thermocouples will be guided to the following positions: right and left neurovascular bundles laterally; external sphincter inferiorly, apex; and posterior prostate at the prostato-rectal interface. Placing of the thermocouples, which supply real-time data to the operator via the cryoablation control unit, allows for objective data to be collected related to actual intraprostatic temperatures achieved. By providing real-time feedback to the operating console during the freezing process, the thermocouples also assist in preventing the operator from freezing through structures - such as the anterior rectal wall - during cryoablation of the prostate. A warming catheter circulating irrigant maintained at 40°C will protect the urethral lining from destructive temperatures. Once core body temperature is achieved as demonstrated by thermocouple temperature reading, a second "freeze/thaw" cycle will be undertaken, thus accomplishing a double freeze/thaw cryotreatment of the prostate. Cryoablation will end with the attainment of 37°C in the previously frozen prostate following the second freeze/thaw cycle. At that time, the autologous dendritic cell injection(s) will be performed as described in Example 3.

### Example 3 - Autologous dendritic cell administration

An individualized Treatment Plan will be prepared for each subject. The Treatment Plan will envision the prostate in four quadrants from a coronal perspective: right superior (RS), right inferior (RI), left superior (LS), and left inferior (LI). Using the RS/RI/LS/LI quadrant system and most recently obtained prostatic biopsy, the investigator will grade each quadrant on a scale of: - No cancer present; + Low volume of cancer in the quadrant; (<50% index tumour volume) ++ Intermediate volume of cancer in the quadrant; (=50% index tumour volume) +++ High volume of cancer in the quadrant. (Index tumour location).

The number of DCs to be delivered to each of the quadrants will be proportionate to the relative estimated volume of disease residing there. For instance, in the case that the RS quadrant is graded as (+++), and the LS quadrant is graded as (++), with the other two quadrants not exhibiting disease, 60% (3/5) of the cell volume will be administered to the RS quadrant and 40% (2/5) of the cell volume will be administered to the LS quadrant (e.g. 2.5 x 10⁷ dose in 1ml; 0.6ml injected to RS and 0.4 ml to LS quadrant, respectively). This proportional approach will hold for all cell doses delivered.

Frozen DC prepared in Example 1 will be placed into a 37°C water bath for thawing. Thawing will be noted as the disappearance of ice crystals from the interior of the cryovial while in the water bath. Once thawed the vial contents will be drawn into a sterile 1ml tuberculin syringe fitted with an 18 gauge, 1.5 inch drawing-up needle. Once the vial contents have been drawn into the syringe, the operator remove the drawing-up needle, and attach to the syringe an 18 gauge, 20 cm Chiba® biopsy needle (Cook Medical, Inc., Bloomington, Indiana). Referring to the Treatment Plan, the operator will insert the needle into the prostate gland under ultrasound guidance until the base of the prostate is reached by the needle tip. Using the quadrant system described he/she will begin with the RS quadrant (if appropriate) and move clockwise to all involved quadrants. Following localization of the needle tip to the prostate base as confirmed by ultrasound imaging, he/she will depress the plunger and slowly withdraw the syringe/needle complex, depositing cells along the length of the quadrant. In the event that a large lesion is noted at diagnosis in a particular location within the quadrant - for instance, where cancer has been noted at the base, but not the mid-gland or apex, he/she may elect to preferentially inject cells at that location. Approximately 0.3 ml of cell suspension will remain in the needle following the injection. Leaving the needle in place, the physician-operator will remove the now-empty syringe used for cell administration and replace with a syringe containing saline solution. He/she will flush the needle by injecting 0.3ml of saline solution into the needle, thereby ensuring the administration of the entire dosage of DCs. Each 1.0 ml vial of dendritic cells with be injected using a 1.0 ml tuberculin syringe. This process will be repeated in accordance with the Treatment Plan, until all cell vials have been deposited in the thawed, post-cryoablation prostate.

### Example 4 - Local (intratumoural) and systemic (i.v.) administration of immune cell checkpoint inhibitors

The immune checkpoint inhibitor ipilimumab (Yervoy®)) is a fully human anti-CTLA-4 monoclonal antibody (IgG1κ) produced in Chinese hamster ovary cells by recombinant DNA technology. Each ml of concentrate contains 5 mg ipilimumab. The solution is clear to slightly opalescent, colourless to pale yellow liquid that may contain light (few) particulates and has a pH of 7.0 and an osmolarity of 260-300 mOsm/kg. Each ml of concentrate contains 0.1 mmol sodium, which is 2.3 mg sodium. One 10 ml vial contains 50 mg of ipilimumab. One 40 ml vial contains 200 mg ipilimumab.

An individualized Treatment Plan will be prepared for each subject in order to supplement the cryoimmunotherapy procedure of Examples 1 to 3 with the immune checkpoint inhibitor ipilimumab. Several patients will be administered ipilimumab at 0.3 mg/kg bodyweight locally and several patients will be administered ipilimumab at 0.6 mg/kg bodyweight locally. The maximum administered dose will be limited to 50 mg irrespective of bodyweight.

The autologous dendritic cell administration will first be performed as described in Example 3. Immediately upon completion of the intratumoral administration of autologous dendritic cells one vial of 10 ml ipilimumab 5 mg/ml (Yervoy®) will be presented to the urologist by the assistant nurse and the required volume, restricted to maximum 50mg (10ml) will be drawn into a sterile 10 ml Braun Omnifix syringe using a BD Microlance 18 gauge, 1.5 inch drawing needle. Once the vial contents have been drawn into the syringe, the operator will remove the drawing-up needle, and attach to the syringe an 18 gauge, 20 cm Chiba® biopsy needle (Cook Medical, Inc., Bloomington, Indiana). Referring to the Treatment Plan, the operator will insert the needle into the ablated prostate gland under ultrasound guidance until the base of the prostate is reached by the needle tip. Thereafter the volume of ipilimumab in the syringe will be distributed slowly (about 1 ml per minute) into the cryoablated area following the same injection routes as used for injection of autologous dendritic cells. It is acceptable if not the entire volume is deposited into the cryoablated area, but additionally into neighbouring non-necrotic tissue.

Several other patients following cyroablation will receive one single intravenous administration of ipilimumab at a cumulative dose of 3 mg/kg bodyweight. The total dose will be diluted to 100 ml using sterile 0.9 % NaCl and thereafter transferred to an empty PVC or non-PVC IV bag and infused over 90 minutes. A DEHP-free and latex-free IV set with a 0.2 micron in-line filter will be used.

### Example 5 - Optional concomitant low-dose cyclophosphamide therapy for selective TReg depletion

The following regimen for TReg depletive therapy will be undertaken if required.

At three days from cryoablation and DC administration 300 mg/m² cyclophosphamide (Cy) will be administered by intravenous infusion (IV), (Berd et al., 1987, Cancer Research 47:3317-3321). Starting at week 2 from cryoablation and DC administration, 25 mg Cy, p.o., b.i.d. will be administered for 7 days on and then administration will cease for 7 days before restarting for 7 days. A further 7 days without Cy administration will follow. This 28 day cycle will be repeated 6 times. The effects of Cy will be monitored by measuring circulating levels of TReg cells (i.e. CD4+ CD25+ Foxp3+ T cells) in the subject's blood, as described extensively in in Ghiringhelli et al., 2007, Cancer Immunology, Immunotherapy 56:641-648).

### Example 6 - Diagnostic imaging

Disease progression and treatment effects will be assessed by *in vivo* radiology and nuclear medicine imaging, i.e. magnetic resonance imaging (MRI), contrast enhanced computer tomography (diagnostic CT), single photon emission computer tomographic (SPECT) and positron emission tomography (PET). The following imaging schedule will be performed in each patient:

### Inclusion /exclusion

| Procedure | Purpose | Sequence/tracer | Time |
|---|---|---|---|
| Prostate MRI | Staging of local disease | T2W, DWI, DCE-MRI | 40 min |
| MRI columna/Pelvis | Nodal/bone metastasis | T1W/STIR | 15 min |
| SPECT | Bone metastasis | (Tc-99m-MD) | 60 min |
| PET-CT | | | |
| (diagnostic CT) | Metastasis/ primary tumour | FACBC/FDG | 30 min |
| | | | |

| **22 weeks** | | | |
|---|---|---|---|
| Prostate MRI | Restaging of local disease Verification of region treated | T2W, DWI, DCE-MRI | 40 min |
| MRI columna/Pelvis | Nodal/bone metastasis | T1W/STIR | 15 min |
| SPECT | Bone metastasis | (Tc-99m-MD) | 60 min |
| PET-CT | | | |
| (diagnostic CT) | Metastasis/ primary tumour | FACBC/FDG | 30 min |
| | | | |

| **46 weeks** | | | |
|---|---|---|---|
| Prostate MRI | Local recurrence / restaging | T2W, DWI, DCE | 40 min |
| MR columna/Pelvis | Nodal/bone metastasis | T1W/STIR | 15 min |
| SPECT | Bone metastasis | (Tc-99m-MD) | 60 min |
| PET-CT | | | |
| (diagnostic CT) | Metastasis/ primary tumour | FACBC/FDG | 30 min |

Prostate MRI and MRI columna/pelvis is preferably done in same the imaging session, i.e. total scan time of about 60 minutes using a 1.5Tesla MRI system.

Prostate MRI is performed without use of endorectal coil to increase patient compliance and minimize total scan time.

SPECT and PET-CT will be performed on separate imaging days. The CT from the PET-CT examination will be used for RECIST evaluations.

PET: [18F] FACBC will be used as the preferred PET tracer, but if FACBC fails to identify the tumour/metastasis, e.g. in low differentiated tutors, imaging using [18F] FDG will be applied on a separate day.

No individual imaging session will exceed 75 minutes.

### Example 7 - Cytotoxic effects of ipilimumab on dendritic cells

For generation of monocyte derived DC (moDC), 20 to 45 ml heparinized blood were collected from each participant and handled within 2 hours. Peripheral blood mononuclear cells were isolated by gradient centrifugation using Lymphoprep (Axis Shield PoC AS, Oslo, Norway) and monocytes isolated by plastic adherence in X-VIVO 20 medium (Lonza, Verviers, Belgium). Nonadherent cells were removed after 1 hour of incubation at 37°C and 5% carbon dioxide. Remaining monocytes were cultured in RP10 medium (RPMI 1640 with Ultraglutamine (Bio Whittaker, Lonza), 10% FCS Gold (PAA, Pasching, Austria), 1% penicillin-streptomycin (Gibco, Invitrogen Corporation, Paisley, UK)) supplemented with 100 ng/ml granulocyte-macrophage colony-stimulating factor (GM-CSF; ImmunoTools GmbH, Friesoythe, Germany) and 20 ng/ ml IL-4 (ImmunoTools GmbH). After 3 days in culture, maturation was induced in one part of the moDC by adding 0.1 µg/ml TLR4 ligand lipopolysaccharide (LPS) (InvivoGen, San Diego, CA, USA) for 24 hours. Ipilimumab up to 0.5 mg/ml or DMSO was added at the same time as LPS and at the same time to un-treated DCs. Following incubation for 24 hours the cells were harvested for Cytation 5™ imaging system analyses and for flowcytometric analysis using the FITC Annexin V Apoptosis Detection Kit (BD Biosciences Cat. No 556547) following the manufacturer's protocol, or for microscopic analysis.

Results are shown in Figure 1. Microscopic analysis and flowcytometric apoptosis (Annexin V-FITC) or other cell death (propidium iodide) analysis of LPS induced mature DCs and immature DCs show that treatment with up to 0.5 mg/ml ipilimumab for 24 hours cause no detectable cytotoxic effects on DCs. The concentration of ipilimumab used in these studies is much higher than typical clinical doses. These results show, for the first time, that the use of immune cell checkpoint inhibitors together with DCs, e.g. during local co-administration to an at least ablated tumour, would not induce DC toxicity and thus limit the effectiveness of any anti-tumour response.

### Example 8 - Clinical treatment of late stage prostate cancer patients in accordance with the invention

14 late stage prostate cancer (metastatic castration resistant prostate cancer (CRPC)) patients were treated as described in Examples 1 to 4. Patients R01 to R09 received cryoablation and doses of DCs, but not ipilimumab. Patients R10 to R13 received highest dosing of DC and 0.3 mg/kg bodyweight of ipilimumab. Patient R14 received highest dosing of DC and 0.5 mg/kg bodyweight of ipilimumab. At predetermined time points circulating tumour cells (CTC) were enumerated according to the following protocol.

7.5 ml peripheral whole blood was collected in CellSave tubes (Immunicon, Inc., Huntingdon Valley, PA). The semi-automated analysis was performed as described elsewhere. Blood samples were kept at room temperature for ≤72 hours before analysis using the CellSearch™ assay (CellSearch™ Epithelial Cell Kit/CellSpotter™ Analyser, Menarini-Silicon Biosystems, San Diego, CA, USA). The assay uses a ferrofluid coated with antibodies to epithelial cell adhesion molecule (EpCAM) to immunomagnetically separate cells of epithelial origin from blood, and fluorescent staining to differentiate between debris, hematopoietic cells, and epithelial-derived circulating tumour cells. CTCs quantified and characterized in this study were cells with a positive staining for keratins (K) and nuclear DAPI staining, but negative for the pan-leukocyte marker CD45. Results are shown in Table 1.

**Table 1 - CellSearch circulating tumor cell (CTC) enumeration in weeks pre- and post cryoimmunotherapy for patients R01 to R14 (per 7.5 ml peripheral blood sample)**

| | **Pre-treatment** | | **Weeks post-treatment** | | | | | | | | | |
|---|---|---|---|---|---|---|---|---|---|---|---|---|
| | 1 | 2 | 2 | 6 | 10 | 14 | 22 | 30 | 38 | 46 | 52 | 72 |
| R01 | 8 | 13 | 7 | 14 | | 5 | 58 | | | | | |
| R02 | 0 | 0 | 0 | 0 | | 0 | 0 | 0 | 0 | 0 | 0 | 0 |
| R03 | 0 | 1 | 0 | | | 0 | | 0 | 0 | 0 | | |
| R04 | 0 | | 0 | 0 | 0 | 0 | 0 | 0 | | | | |
| R05 | 3 | 1 | 3 | 1 | 0 | 0 | 0 | 0 | 0 | 0 | 0 | 0 |
| R07 | 0 | 1 | 0 | | 0 | 0 | 1 | 0 | 0 | 0 | | |
| R08 | 0 | 0 | 0 | 0 | | | 0 | 0 | | 0 | | |
| R09 | 2 | 31 | 67 | 137 | 6 | 3 | 4 | 9 | 40 | | | |
| R10 | 9 | 7 | 9 | 24 | 12 | 24 | | | | | | |
| R11 | 0 | 0 | | | | 0 | | | | | | |
| R12 | 0 | 0 | 0 | | 0 | 0 | | | | | | |
| R13 | 6 | 14 | 8 | 18 | 2 | | | | | | | |
| R14 | 0 | 1 | | | | | | | | | | |

Adverse side effects were also monitored during treatment and follow up.

Follow-up of patients R01 - R09 previously treated with cryoablation of the prostate tumor followed by subsequent intratumoral DC injection showed remarkably few adverse events. Thus, it may be concluded that both cryoablation was safe without local surgical or freezing associated damage and no serious autoimmune adverse events are associated with DC administration.

According to published literature, systemic treatment with ipilimumab has been associated with high incidence of autoimmune adverse events, and the adverse events have been correlated to total injected dose per kg bodyweight. Serious adverse events include autoimmune colitis, hepatitis, uveitis, hypophysitis and difficult to treat skin disorders (Perez-De-Lis M, et al. 2017, Autoimmune diseases induced by biological agents. A review of 12,731 cases (BIOGEAS Registry), Expert Opin Drug Saf.;16(11):1255-71; and Rijnders M, de Wit R, et al, 2017, Systematic Review of Immune Checkpoint Inhibition in Urological Cancers, Eur Urol.; 72(3):411-23). It is consequently strongly encouraging that no autoimmune adverse events have been observed during follow-up of patients treated with intratumoral injection of ipilimumab in addition to DC-mediated cryoimmunotherapy. Neither was there any negative acute reactions observed as a consequence of ipilimumab and DC intratumoral injections.

Patients R10 to R14 also showed remarkably few surgical side effects, with no instances of infection being reported and low levels of fistula formation and similar local anatomical deformations being observed. It was not clear from the art whether or not tumour cryoablation and local administration of DCs followed by local administration of immune cell checkpoint inhibitors would cause significant surrounding tissue damage and thereby result in adverse surgical side effects. The follow-up results reveal that tumour ablation, e.g. by cryoablation, followed by local use of DCs together with immune cell checkpoint inhibitors is surprisingly well tolerated by patients and not associated with elevated infection rates, fistula formation and similar local anatomical deformations.

Based upon the CTC results shown in Table 1, the treatments of the invention can be considered to result in disease stabilisation in late stage prostate cancer patients, i.e. worsening of the disease is not observed over the follow-up period in any patient. This may be consider a very positive result in view of the lack of adverse effects seen in the treated patients as compared to those expected in the same patients treated systemically with ipilimumab alone.

It should also be noted that the patients used in this study are those with metastatic castration resistant prostate cancer (CRPC), i.e. very late stage cancer. The included patients have had their diagnosis of prostate cancer for extended periods. Patients were included only after their tumor progresses on standard androgen deprivation therapy (ADT). Patients both pre and post chemotherapy were included. The median overall survival of comparable patient groups according to international literature is 18 months even with docetaxel chemotherapy intervention (Schalken J, Fitzpatrick JM. Enzalutamide: Targeting the androgen signalling pathway in metastatic castration-resistant prostate cancer. BJU international. 2015). These patients are necessarily selected because ethical consent for such trials is only given for patients with very serious disease. It is therefore to be expected that efficacy results observed in this study will not be as apparent as would be expected in patients with earlier stage disease. Indeed, there is theoretical and clinical evidence to suggest that the curative potential of immunotherapy is stronger at an earlier stage and with lower total tumor load (Gulley JL, et al, 2011, Impact of tumour volume on the potential efficacy of therapeutic vaccines, Curr Oncol.;18(3):e150-7). It therefore to be expected that the results observed in this trial will be replicated to a greater extent when repeated in patients with earlier stage disease.

Moreover, the stabilisation of disease in patients with such advanced prostate cancer without the adverse side effects associated with ipilimumab may be considered a particular success.

## Claims

1. An immune cell checkpoint inhibitor for use together with an autologous immature dendritic cell in a method for the treatment of a tumour in a subject, said method comprising administering
(i) the autologous immature dendritic cell and
(ii) the immune cell checkpoint inhibitor
to said subject subsequent to the at least partial ablation of said tumour in said subject by cryoablation, hydrothermal ablation, ionising radiation ablation, radioablation, ultrasound ablation, laser ablation, microwave ablation or electroablation, wherein the administration of said autologous immature dendritic cells and said immune cell checkpoint inhibitor is local to the site of the ablated tumour or part thereof, wherein said immune cell checkpoint inhibitor is an affinity macromolecule reagent which binds to at least one immune checkpoint ligand and/or receptor thereof and the binding of said reagent to its target binding site on said immune checkpoint ligand and/or receptor thereof interferes with the interaction between the receptor and the ligand and wherein said immune checkpoint ligand and/or receptor thereof is selected from
(a) CTLA4 and CD80 or CD86,
(b) PD1 and PDL1 or PDL2,
(c) BTLA and HVEM,
(d) KIR and MHC class I or II,
(e) LAG3 and MHC class I or II,
(f) TIM3 and galectin 9,
(g) A2aR and adenosine,
(h) B7-H3,
(i) B7-H4, and
(j) 2B4.

2. An autologous immature dendritic cell for use together with an immune cell checkpoint inhibitor in a method for the treatment of a tumour in a subject, said method comprising administering
(i) the autologous immature dendritic cell and
(ii) the immune cell checkpoint inhibitor to said subject subsequent to the at least partial ablation of said tumour in said subject by cryoablation, hydrothermal ablation, ionising radiation ablation, radioablation, ultrasound ablation, laser ablation, microwave ablation or electroablation, wherein the administration of said autologous immature dendritic cells and said immune cell checkpoint inhibitor is local to the site of the ablated tumour or part thereof, wherein said immune cell checkpoint inhibitor is an affinity macromolecule reagent which binds to at least one immune checkpoint ligand and/or receptor thereof and the binding of said reagent to its target binding site on said immune checkpoint ligand and/or receptor thereof interferes with the interaction between the receptor and the ligand and wherein said immune checkpoint ligand and/or receptor thereof is selected from
(a) CTLA4 and CD80 or CD86,
(b) PD1 and PDL1 or PDL2,
(c) BTLA and HVEM,
(d) KIR and MHC class I or II,
(e) LAG3 and MHC class I or II,
(f) TIM3 and galectin 9,
(g) A2aR and adenosine,
(h) B7-H3,
(i) B7-H4, and
(j) 2B4.

3. A product containing an autologous immature dendritic cell and an immune cell checkpoint inhibitor as a combined preparation for separate, simultaneous or sequential use in a method for the treatment of a tumour in a subject, said method comprising administering
(i) the autologous immature dendritic cell and
(ii) the immune cell checkpoint inhibitor
to said subject subsequent to the at least partial ablation of said tumour in said subject by cryoablation, hydrothermal ablation, ionising radiation ablation, radioablation, ultrasound ablation, laser ablation, microwave ablation or electroablation, wherein the administration of said autologous immature dendritic cells and said immune cell checkpoint inhibitor is local to the site of the ablated tumour or part thereof, wherein said immune cell checkpoint inhibitor is an affinity macromolecule reagent which binds to at least one immune checkpoint ligand and/or receptor thereof and the binding of said reagent to its target binding site on said immune checkpoint ligand and/or receptor thereof interferes with the interaction between the receptor and the ligand and wherein said immune checkpoint ligand and/or receptor thereof is selected from
(a) CTLA4 and CD80 or CD86,
(b) PD1 and PDL1 or PDL2,
(c) BTLA and HVEM,
(d) KIR and MHC class I or II,
(e) LAG3 and MHC class I or II,
(f) TIM3 and galectin 9,
(g) A2aR and adenosine,
(h) B7-H3,
(i) B7-H4, and
(j) 2B4.

4. The immune cell checkpoint inhibitor for use, autologous immature DC for use, or the product for use of any one of claims 1 to 3, wherein said immune cell checkpoint inhibitor is a protein.

5. The immune cell checkpoint inhibitor for use, autologous immature DC for use, or the product for use of any one of claims 1 to 4, wherein said immune cell checkpoint inhibitor comprises a portion of the amino acid sequence of the checkpoint ligand and/or receptor thereof.

6. The immune cell checkpoint inhibitor for use, autologous immature DC for use, or the product for use of any one of claims 1 to 4, wherein said immune cell checkpoint inhibitor is an antibody, a phage display antibody or an antibody mimetic, preferably , wherein said antibody, phage display antibody or antibody mimetic binds at least one immune checkpoint ligand and/or receptor thereof selected from CTLA4, CD80, CD86, PD1, PDL1, PDL2, LAG3, B7-H3, B7-H4, or TIM3, preferably CTLA4, PD1, PDL1, LAG3 or B7-H3.

7. The immune cell checkpoint inhibitor for use, autologous immature DC for use, or the product for use of any one of claims 1 to 6, wherein said immune cell checkpoint inhibitor is selected from ipilimumab, tremelimumab, nivolumab, pembrolizumab , CT-011, AMP-224 , MDX-1105, RG7446, BMS-936559, MGA271, atezolizumab, avelumab and durvalumab.

8. The immune cell checkpoint inhibitor for use, autologous immature DC for use, or the product for use of any one of claims 1 to 7, wherein said method further comprises prior to administration of the DCs:
(a) leukapheresis of the subject to isolate leukocytes or mononuclear cells from the blood of the subject and/or to isolate monocytes from the blood of the subject,
(b) optionally, isolation of monocytes from the isolated leukocytes or mononuclear cells of (a), and
(c) culture of said monocytes under conditions which permit differentiation of DCs therefrom.

9. The immune cell checkpoint inhibitor for use, autologous immature DC for use, or the product for use of any one of claims 1 to 8, wherein said method comprises a step prior to the administration of the immune cell checkpoint inhibitor and the autologous immature DCs of at least partial tumour ablation by cryoablation, hydrothermal ablation, ionising radiation ablation, radioablation, ultrasound ablation, laser ablation, microwave ablation or electroablation,.

10. The immune cell checkpoint inhibitor for use, autologous immature DC for use, or the product for use of any one of claims 1 to 9, wherein said cryoablation comprises a plurality of freeze/thaw cycles, preferably a freeze/thaw cycle of freezing to less than or equal to about -40°C and warming to about 37°C.

11. The immune cell checkpoint inhibitor for use, autologous immature DC, or the product for use of any one of claims 1 to 10, wherein said tumour is a colorectal tumour, prostate tumour, testicular tumour, skin tumour, breast tumour, kidney tumour, ovarian tumour, stomach tumour, intestinal tumour, liver tumour, pancreatic tumour, lung tumour, oesophageal tumour, oral tumour, throat tumour, brain tumour, adrenal tumour, thyroid tumour, uterine tumour or haematological tumour.

12. The immune cell checkpoint inhibitor for use, autologous immature DC for use, or the product for use of claim 11, wherein said tumour is a cancer, preferably prostate cancer.

13. The, immune cell checkpoint inhibitor for use, autologous immature DC for use, or the product for use of any one of claims 1 to 12, wherein a further pharmaceutical useful in the treatment of neoplastic disease is administered together with the immune cell checkpoint inhibitor and the autologous immature DCs.

14. The product for use of claim 13, said product further containing the further pharmaceutical useful in the treatment of neoplastic disease as a combined preparation for separate, simultaneous or sequential use in said method.

15. The immune cell checkpoint inhibitor for use, autologous immature DC for use of claim 13 or the product for use of claim 14, wherein said further pharmaceutical useful in the treatment of neoplastic disease is selected from a cytotoxic chemotherapy agent, an anti-hormonal agent, an angiogenesis inhibitor, an anticancer monoclonal antibody, a radioimmunotherapeutic, a cancer treatment vaccine, an immunostimulatory agent or an immunosuppressant preferably cyclophosphamide or a prodrug or metabolite thereof, preferably 4-hydroxy cyclophosphamide and N,N-bis(2-chloroethyl)phosphorodiamidic acid.

## Patentansprüche

1. Immunzellen-Checkpoint-Inhibitor zur Verwendung zusammen mit einer autologen unreifen dendritischen Zelle in einem Verfahren zur Behandlung eines Tumors in einem Subjekt, wobei das Verfahren umfasst Verabreichung
(i) der autologen unreifen dendritischen Zelle und
(ii) des Immunzellen-Checkpoint-Inhibitors
an das Subjekt nach der mindestens teilweisen Ablation des Tumors in dem Subjekt durch Kryoablation, hydrothermale Ablation, Ablation durch ionisierende Strahlung, Hochfrequenzablation, Ultraschallablation, Laserablation, Mikrowellenablation oder Elektroablation, wobei die Verabreichung der autologen unreifen dendritischen Zellen und des Immunzellen-Checkpoint-Inhibitors lokal an der Stelle des abgetragenen Tumors oder eines Teils davon erfolgt, wobei der Immunzellen-Checkpoint-Inhibitor ein Affinitätsmakromolekülreagens ist, das sich an mindestens einen Immunzellen-Checkpoint-Liganden und/oder Rezeptor davon bindet und die Bindung des Reagenz an seine Target-Bindestelle auf dem Immunzellen-Checkpoint-Liganden und/oder Rezeptor davon die Interaktion zwischen dem Rezeptor und dem Liganden beeinträchtigt und wobei der Immunzellen-Checkpoint-Ligand und/oder Rezeptor davon ausgewählt ist aus
(a) CTLA4 und CD80 oder CD86,
(b) PD1 und PDL1 oder PDL2,
(c) BTLA und HVEM,
(d) KIR und MHC-Klasse I oder II,
(e) LAG3 und MHC-Klasse I oder II,
(f) TIM3 und Galectin 9,
(g) A2aR und Adenosin,
(h) B7-H3,
(i) B7-H4, und
(j) 2B4.

2. Autologe unreife dendritische Zelle zur Verwendung zusammen mit einem Immunzellen-Checkpoint-Inhibitor in einem Verfahren zur Behandlung eines Tumors in einem Subjekt, wobei das Verfahren umfasst Verabreichung
(i) der autologen unreifen dendritischen Zelle und
(ii) des Immunzellen-Checkpoint-Inhibitors
an das Subjekt nach der mindestens teilweisen Ablation des Tumors in dem Subjekt durch Kryoablation, hydrothermale Ablation, Ablation durch ionisierende Strahlung, Hochfrequenzablation, Ultraschallablation, Laserablation, Mikrowellenablation oder Elektroablation, wobei die Verabreichung der autologen unreifen dendritischen Zellen und des Immunzellen-Checkpoint-Inhibitors lokal an der Stelle des abgetragenen Tumors oder eines Teils davon erfolgt, wobei der Immunzellen-Checkpoint-Inhibitor ein Affinitätsmakromolekülreagens ist, das sich an mindestens einen Immunzellen-Checkpoint-Liganden und/oder Rezeptor davon bindet und die Bindung des Reagenz an seine Target-Bindestelle auf dem Immunzellen-Checkpoint-Liganden und/oder Rezeptor davon die Interaktion zwischen dem Rezeptor und dem Liganden beeinträchtigt und wobei der Immunzellen-Checkpoint-Ligand und/oder Rezeptor davon ausgewählt ist aus
(a) CTLA4 und CD80 oder CD86,
(b) PD1 und PDL1 oder PDL2,
(c) BTLA und HVEM,
(d) KIR und MHC-Klasse I oder II,
(e) LAG3 und MHC-Klasse I oder II,
(f) TIM3 und Galectin 9,
(g) A2aR und Adenosin,
(h) B7-H3,
(i) B7-H4, und
(j) 2B4.

3. Produkt, das eine autologe unreife dendritische Zelle und einen Immunzellen-Checkpoint-Inhibitor als ein kombiniertes Präparat zur getrennten, gleichzeitigen oder aufeinanderfolgenden Verwendung in einem Verfahren zur Behandlung eines Tumors in einem Subjekt enthält, wobei das Verfahren umfasst Verabreichung
(i) der autologen unreifen dendritischen Zelle und
(ii) des Immunzellen-Checkpoint-Inhibitors
an das Subjekt nach der mindestens teilweisen Ablation des Tumors in dem Subjekt durch Kryoablation, hydrothermale Ablation, Ablation durch ionisierende Strahlung, Hochfrequenzablation, Ultraschallablation, Laserablation, Mikrowellenablation oder Elektroablation, wobei die Verabreichung der autologen unreifen dendritischen Zellen und des Immunzellen-Checkpoint-Inhibitors lokal an der Stelle des abgetragenen Tumors oder eines Teils davon erfolgt, wobei der Immunzellen-Checkpoint-Inhibitor ein Affinitätsmakromolekülreagens ist, das sich an mindestens einen Immunzellen-Checkpoint-Liganden und/oder Rezeptor davon bindet und die Bindung des Reagenz an seine Target-Bindestelle auf dem Immunzellen-Checkpoint-Liganden und/oder Rezeptor davon die Interaktion zwischen dem Rezeptor und dem Liganden beeinträchtigt und wobei der Immunzellen-Checkpoint-Ligand und/oder Rezeptor davon ausgewählt ist aus
(a) CTLA4 und CD80 oder CD86,
(b) PD1 und PDL1 oder PDL2,
(c) BTLA und HVEM,
(d) KIR und MHC-Klasse I oder II,
(e) LAG3 und MHC-Klasse I oder II,
(f) TIM3 und Galectin 9,
(g) A2aR und Adenosin,
(h) B7-H3,
(i) B7-H4, und
(j) 2B4.

4. Immunzellen-Checkpoint-Inhibitor zur Verwendung, autologe unreife DZ zur Verwendung oder Produkt zur Verwendung nach einem der Ansprüche 1 bis 3, wobei der Immunzellen-Checkpoint-Inhibitor ein Protein ist.

5. Immunzellen-Checkpoint-Inhibitor zur Verwendung, autologe unreife DZ zur Verwendung oder Produkt zur Verwendung nach einem der Ansprüche 1 bis 4, wobei der Immunzellen-Checkpoint-Inhibitor einen Abschnitt der Aminosäuresequenz des Immunzellen-Checkpoint-Liganden und/oder Rezeptors davon umfasst.

6. Immunzellen-Checkpoint-Inhibitor zur Verwendung, autologe unreife DZ zur Verwendung oder Produkt zur Verwendung nach einem der Ansprüche 1 bis 4, wobei der Immunzellen-Checkpoint-Inhibitor ein Antikörper, ein Antikörper im Phagen-Display oder ein Antikörpermimetikum ist, wobei vorzugsweise der Antikörper, der Antikörper im Phagen-Display oder das Antikörpermimetikum sich mit mindestens einem Immunzellen-Checkpoint-Liganden und/oder Rezeptor davon bindet, ausgewählt aus CTLA4, CD80, CD86, PD1, PDL1, PDL2, LAG3, B7-H3, B7-H4 oder TIM3, vorzugsweise CTLA4, PD1, PDL1, LAG3 oder B7-H3.

7. Immunzellen-Checkpoint-Inhibitor zur Verwendung, autologe unreife DZ zur Verwendung oder Produkt zur Verwendung nach einem der Ansprüche 1 bis 6, wobei der Immunzellen-Checkpoint-Inhibitor ausgewählt ist aus Ipilimumab, Tremelimumab, Nivolumab, Pembrolizumab, CT-011, AMP-224, MDX-1105, RG7446, BMZ-936559, MGA271 Atelzolizumab, Avelumab und Durvalumab.

8. Immunzellen-Checkpoint-Inhibitor zur Verwendung, autologe unreife DZ zur Verwendung oder Produkt zur Verwendung nach einem der Ansprüche 1 bis 7, wobei das Verfahren weiter vor Verabreichung der DZn umfasst:
(a) Leukapherese des Subjekts, um Leukozyten oder mononukleare Zellen aus dem Blut des Subjekts zu isolieren und/oder um Monozyten aus dem Blut des Subjekts zu isolieren,
(b) wahlweise, Isolierung von Monozyten aus den isolierten Leukozyten oder mononuklearen Zellen von (a), und
(c) Kultivierung der Monozyten unter Bedingungen, die eine Differenzierung von DZn daraus ermöglichen.

9. Immunzellen-Checkpoint-Inhibitor zur Verwendung, autologe unreife DZ zur Verwendung oder Produkt zur Verwendung nach einem der Ansprüche 1 bis 8, wobei das Verfahren vor der Verabreichung des Immunzellen-Checkpoint-Inhibitors und der autologen unreifen DZn einen Schritt einer mindestens teilweisen Tumorablation durch Kryoablation, hydrothermale Ablation, Ablation durch ionisierende Strahlung, Hochfrequenzablation, Ultraschallablation, Laserablation, Mikrowellenablation oder Elektroablation umfasst.

10. Immunzellen-Checkpoint-Inhibitor zur Verwendung, autologe unreife DZ zur Verwendung oder Produkt zur Verwendung nach einem der Ansprüche 1 bis 9, wobei die Kryoablation eine Vielzahl von Herunterkühlen-Auftauzyklen, vorzugsweise einen Herunterkühlen-Auftauzyklus des Herunterkühlens auf weniger oder gleich etwa -40 °C und Erwärmens auf etwa 37 °C, umfasst.

11. Immunzellen-Checkpoint-Inhibitor zur Verwendung, autologe unreife DZ zur Verwendung oder Produkt zur Verwendung nach einem der Ansprüche 1 bis 10, wobei der Tumor ein kolorektaler Tumor, Prostatatumor, Hodentumor, Hauttumor, Brusttumor, Nierentumor, Eierstocktumor, Magentumor, Darmtumor, Lebertumor, Bauchspeicheldrüsentumor, Lungentumor, Speiseröhrentumor, Mundhöhlentumor, Kehltumor, Hirntumor, Nebennierentumor, Schilddrüsentumor, Gebärmuttertumor oder Bluttumor ist.

12. Immunzellen-Checkpoint-Inhibitor zur Verwendung, autologe unreife DZ zur Verwendung oder Produkt zur Verwendung nach Anspruch 11, wobei der Tumor ein Krebs ist, vorzugsweise Prostatakrebs.

13. Immunzellen-Checkpoint-Inhibitor zur Verwendung, autologe unreife DZ zur Verwendung oder Produkt zur Verwendung nach einem der Ansprüche 1 bis 12, wobei ein weiteres Arzneimittel, das bei der Behandlung neoplastischer Erkrankung nützlich ist, zusammen mit dem Immunzellen-Checkpoint-Inhibitor und den autologen unreifen DZn verabreicht wird.

14. Produkt zur Verwendung nach Anspruch 13, wobei das Produkt weiter das weitere, bei der Behandlung neoplastischer Erkrankung nützliche Arzneimittel als ein kombiniertes Präparat zur getrennten, gleichzeitigen oder aufeinanderfolgenden Verwendung in dem Verfahren enthält.

15. Immunzellen-Checkpoint-Inhibitor zur Verwendung, autologe unreife DZ zur Verwendung nach Anspruch 13 oder Produkt zur Verwendung nach Anspruch 14, wobei das weitere, bei der Behandlung neoplastischer Erkrankung nützliche Arzneimittel ausgewählt ist aus einem zytotoxischen Chemotherapie-Wirkstoff, einem antihormonellen Wirkstoff, einem Angiogenese-Inhibitor, einem krebsbekämpfenden monoklonalen Antikörper, einem Radioimmuntherapeutikum, einem Krebsbehandlungsimpfstoff, einem immunstimulierenden Wirkstoff oder einem Immunsuppressivum, vorzugsweise einem Cyclphosphamid oder einem Prodrug oder Metaboliten davon, vorzugsweise 4-Hydroxycyclophosphamid und N,N-Bis(2-Chlorethyl)phosphordiamidsäure.

## Revendications

1. Inhibiteur de point de contrôle de cellule immunitaire pour une utilisation conjointement avec une cellule dendritique immature autologue dans un procédé pour le traitement d'une tumeur chez un sujet, ledit procédé comprenant l'administration
(i) de la cellule dendritique immature autologue et
(ii) de l'inhibiteur de point de contrôle de cellule immunitaire
audit sujet à la suite de l'ablation au moins partielle de ladite tumeur chez ledit sujet par cryoablation, ablation hydrothermique, ablation par rayonnements ionisants, ablation par radiofréquence, ablation par ultrasons, ablation laser, ablation par micro-ondes ou électroablation, dans lequel l'administration desdites cellules dendritiques immatures autologues et dudit inhibiteur de point de contrôle de cellule immunitaire est locale sur le site de la tumeur ablatée ou une partie de celui-ci, dans lequel ledit inhibiteur de point de contrôle de cellule immunitaire est un réactif de macromolécule d'affinité qui se lie à au moins un ligand de point de contrôle immunitaire et/ou un récepteur de celui-ci et la liaison dudit réactif avec son site de liaison cible sur lesdits ligand de point de contrôle immunitaire et/ou récepteur de celui-ci interfère avec l'interaction entre le récepteur et le ligand et dans lequel lesdits ligand de point de contrôle immunitaire et/ou récepteur de celui-ci sont sélectionnés parmi
a) CTLA4 et CD80 ou CD86,
b) PD1 et PDL1 ou PDL2,
c) BTLA et HEVM,
d) KIR et MHC de classe I ou II,
e) LAG3 et MHC de classe I ou II,
f) TIM3 et la galectine 9,
g) A2aR et l'adénosine,
h) B7-H3,
i) B7-H4, et
j) 2B4.

2. Cellule dendritique immature autologue pour une utilisation conjointement avec un inhibiteur de point de contrôle de cellule immunitaire dans un procédé pour le traitement d'une tumeur chez un sujet, ledit procédé comprenant l'administration
(i) de la cellule dendritique immature autologue et
(ii) de l'inhibiteur de point de contrôle de cellule immunitaire
audit sujet à la suite de l'ablation au moins partielle de ladite tumeur chez ledit sujet par cryoablation, ablation hydrothermique, ablation par rayonnements ionisants, ablation par radiofréquence, ablation par ultrasons, ablation laser, ablation par micro-ondes ou électroablation, dans lequel l'administration desdites cellules dendritiques immatures autologues et dudit inhibiteur de point de contrôle de cellule immunitaire est locale sur le site de la tumeur ablatée ou une partie de celui-ci, dans lequel ledit inhibiteur de point de contrôle de cellule immunitaire est un réactif de macromolécule d'affinité qui se lie à au moins un ligand de point de contrôle immunitaire et/ou un récepteur de celui-ci et la liaison dudit réactif avec son site de liaison cible sur lesdits ligand de point de contrôle immunitaire et/ou récepteur de celui-ci interfère avec l'interaction entre le récepteur et le ligand et dans lequel lesdits ligand de point de contrôle immunitaire et/ou récepteur de celui-ci sont sélectionnés parmi
a) CTLA4 et CD80 ou CD86,
b) PD1 et PDL1 ou PDL2,
c) BTLA et HEVM,
d) KIR et MHC de classe I ou II,
e) LAG3 et MHC de classe I ou II,
f) TIM3 et la galectine 9,
g) A2aR et l'adénosine,
h) B7-H3,
i) B7-H4, et
j) 2B4.

3. Produit contenant une cellule dendritique immature autologue et un inhibiteur de point de contrôle de cellule immature en tant que préparation combinée pour une utilisation séparée, simultanée ou séquentielle dans un procédé pour le traitement d'une tumeur chez un sujet, ledit procédé comprenant l'administration
(i) de la cellule dendritique immature autologue et
(ii) de l'inhibiteur de point de contrôle de cellule immunitaire
audit sujet à la suite de l'ablation au moins partielle de ladite tumeur chez ledit sujet par cryoablation, ablation hydrothermique, ablation par rayonnements ionisants, ablation par radiofréquence, ablation par ultrasons, ablation laser, ablation par micro-ondes ou électroablation, dans lequel l'administration desdites cellules dendritiques immatures autologues et dudit inhibiteur de point de contrôle de cellule immunitaire est locale sur le site de la tumeur ablatée ou une partie de celui-ci, dans lequel ledit inhibiteur de point de contrôle de cellule immunitaire est un réactif de macromolécule d'affinité qui se lie à au moins un ligand de point de contrôle immunitaire et/ou un récepteur de celui-ci et la liaison dudit réactif avec son site de liaison cible sur lesdits ligand de point de contrôle immunitaire et/ou récepteur de celui-ci interfère avec l'interaction entre le récepteur et le ligand et dans lequel lesdits ligand de point de contrôle immunitaire et/ou récepteur de celui-ci sont sélectionnés parmi
a) CTLA4 et CD80 ou CD86,
b) PD1 et PDL1 ou PDL2,
c) BTLA et HEVM,
d) KIR et MHC de classe I ou II,
e) LAG3 et MHC de classe I ou II,
f) TIM3 et la galectine 9,
g) A2aR et l'adénosine,
h) B7-H3,
i) B7-H4, et
j) 2B4.

4. Inhibiteur de point de contrôle de cellule immunitaire pour une utilisation, CD immature autologue pour une utilisation ou produit pour une utilisation selon l'une quelconque des revendications 1 à 3, dans lesquels ledit inhibiteur de point de contrôle de cellule immunitaire est une protéine.

5. Inhibiteur de point de contrôle de cellule immunitaire pour une utilisation, CD immature autologue pour une utilisation ou produit pour une utilisation selon l'une quelconque des revendications 1 à 4, dans lesquels ledit inhibiteur de point de contrôle de cellule immunitaire comprend une partie de la séquence d'acides aminés du ligand de point de contrôle et/ou récepteur de celui-ci.

6. Inhibiteur de point de contrôle de cellule immunitaire pour une utilisation, CD immature autologue pour une utilisation ou produit pour une utilisation selon l'une quelconque des revendications 1 à 4, dans lesquels ledit inhibiteur de point de contrôle de cellule immunitaire est un anticorps, un anticorps d'exposition sur phage ou un mimétique d'anticorps, de préférence, dans lesquels ledit anticorps, anticorps d'exposition sur phage ou mimétique d'anticorps se lie à au moins un ligand de point de contrôle immunitaire et/ou récepteur de celui-ci sélectionné parmi CTLA4, CD80, CD86, PD1, PDL1, PDL2, LAG3, B7-H3, B7-H4 ou TIM3, de préférence CTLA4, PD1, PDL1, LAG3 ou B7-H3.

7. Inhibiteur de point de contrôle de cellule immunitaire pour une utilisation, CD immature autologue pour une utilisation ou produit pour une utilisation selon l'une quelconque des revendications 1 à 6, dans lesquels ledit inhibiteur de point de contrôle de cellule immunitaire est sélectionné parmi l'ipilimumab, le trémélimumab, le nivolumab, le pembrolizumab, CT-011, AMP-224, MDX-1105, RG7446, BMS-936559, MGA271, l'atézolizumab, l'avélumab et le durvalumab.

8. Inhibiteur de point de contrôle de cellule immunitaire pour une utilisation, CD immature autologue pour une utilisation ou produit pour une utilisation selon l'une quelconque des revendications 1 à 7, dans lesquels ledit procédé comprend en outre avant l'administration des CD :
(a) une leucaphérèse du sujet pour isoler des leucocytes ou des cellules mononucléaires du sang du sujet et/ou pour isoler des monocytes du sang du sujet,
(b) facultativement, un isolement de monocytes des leucocytes isolés ou des cellules mononucléaires de (a), et
(c) une culture desdits monocytes dans des conditions qui permettent une différenciation des CD originaires de ceux-ci.

9. Inhibiteur de point de contrôle de cellule immunitaire pour une utilisation, CD immature autologue pour une utilisation ou produit pour une utilisation selon l'une quelconque des revendications 1 à 8, dans lesquels ledit procédé comprend une étape avant l'administration de l'inhibiteur de point de contrôle de cellule immunitaire et des CD immatures autologues d'au moins une ablation de tumeur partielle par cryoablation, ablation hydrothermique, ablation par rayonnement ionisant, ablation par radiofréquence, ablation par ultrasons, ablation laser, ablation par micro-ondes ou électroablation.

10. Inhibiteur de point de contrôle de cellule immunitaire pour une utilisation, CD immature autologue pour une utilisation ou produit pour une utilisation selon l'une quelconque des revendications 1 à 9, dans lesquels ladite cryoablation comprend une pluralité de cycles de congélation/décongélation, de préférence un cycle de congélation/décongélation de congélation inférieure ou égale à environ -40 °C et de réchauffement à environ 37 °C.

11. Inhibiteur de point de contrôle de cellule immunitaire pour une utilisation, CD immature autologue ou produit pour une utilisation selon l'une quelconque des revendications 1 à 10, dans lesquels ladite tumeur est une tumeur colorectale, une tumeur de la prostate, un tumeur des testicules, une tumeur de la peau, une tumeur du sein, une tumeur du rein, une tumeur de l'ovaire, une tumeur de l'estomac, une tumeur intestinale, une tumeur du foie, une tumeur du pancréas, une tumeur du poumon, une tumeur œsophagienne, une tumeur de la bouche, une tumeur de la gorge, une tumeur du cerveau, une tumeur surrénale, une tumeur thyroïdienne, une tumeur utérine ou une tumeur hématologique.

12. Inhibiteur de point de contrôle de cellule immunitaire pour une utilisation, CD immature autologue pour une utilisation ou produit pour une utilisation selon la revendication 11, dans lesquels ladite tumeur est un cancer, de préférence un cancer de la prostate.

13. Inhibiteur de point de contrôle de cellule immunitaire pour une utilisation, CD immature autologue pour une utilisation ou produit pour une utilisation selon l'une quelconque des revendications 1 à 12, dans lesquels un agent pharmaceutique supplémentaire utile dans le traitement d'une maladie néoplasique est administré conjointement avec l'inhibiteur de point de contrôle de cellule immunitaire et les CD immatures autologues.

14. Produit pour une utilisation selon la revendication 13, ledit produit contenant en outre l'agent pharmaceutique supplémentaire utile dans le traitement d'une maladie néoplasique en tant que préparation combinée pour une utilisation séparée, simultanée ou séquentielle dans ledit procédé.

15. Inhibiteur de point de contrôle de cellule immunitaire pour une utilisation, CD immature autologue pour une utilisation selon la revendication 13 ou produit pour une utilisation selon la revendication 14, dans lesquels ledit agent pharmaceutique supplémentaire utile dans le traitement d'une maladie néoplasique est sélectionné parmi un agent chimiothérapeutique cytotoxique, un agent antihormonal, un inhibiteur de l'angiogenèse, un anticorps monoclonal anticancéreux, un agent radioimmunothérapeutique, un vaccin de traitement du cancer, un agent immunostimulant ou un immunosuppresseur de préférence le cyclophosphamide ou un promédicament ou un métabolite de celui-ci, de préférence le 4-hydroxy-cyclophosphamide et l'acide N,N-bis(2-chloroéthyl)phosphoradiamidique.
